(19) **Europäisches Patentamt**
**European Patent Office**
**Office européen des brevets**

(11) **EP 1 513 092 B1**

(12) **EUROPÄISCHE PATENTSCHRIFT**

(45) Veröffentlichungstag und Bekanntmachung des Hinweises auf die Patenterteilung:
**24.06.2009 Patentblatt 2009/26**

(51) Int Cl.:
***G06F 19/00*** *(2006.01)*

(21) Anmeldenummer: **03020301.2**

(22) Anmeldetag: **08.09.2003**

(54) **Verfahren zur Konformationsanalyse von Aminosäuresequenzen**

Method for the analysis of amino acid sequence conformations

Méthode d'analyse de conformations des séquences d'amino-acides

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**09.03.2005 Patentblatt 2005/10**

(73) Patentinhaber: **ACGT Progenomics AG**
**06120 Halle (Saale) (DE)**

(72) Erfinder:
• **Dallüge, Roman**
**04109 Leipzig (DE)**
• **Böhm, Gerald**
**06114 Halle (Saale) (DE)**

(74) Vertreter: **Reinhard - Skuhra - Weise & Partner GbR**
**Patent- und Rechtsanwälte**
**P.O. Box 440151**
**80750 München (DE)**

(56) Entgegenhaltungen:
• **S.SUDARSANAM, S.SRINIVASAN: "Sequence-dependent conformational sampling using a database of phi i+1 and psi i angles for predicting polypeptide backbone conformations" PROTEIN ENGINEERING, Bd. 10, Nr. 10, November 1997 (1997-11), Seiten 1155-1162, XP002269794**
• **H.S.KANG, N.A.KUROCHKINA, B.LEE: "Estimation and use of Protein Backbone Angle Probabilities" J. MOL. BIOL., Bd. 229, Nr. 2, 20. Januar 1993 (1993-01-20), Seiten 448-460, XP002269795**
• **J.-F.GIBRAT, B.ROBSON, J.GARNIER: "Influence of the Local Amino Acid Sequence upon the Zones of the Torsional Angles Phi and Psi Adopted by Residues in Proteins" BIOCHEMISTRY, Bd. 30, Nr. 6, Februar 1991 (1991-02), Seiten 1578-1586, XP002269796**
• **NEEDLEMAN S B ET AL: "A GENERAL METHOD APPLICABLE TO THE SEARCH FOR SIMILARITIES IN THE AMINO ACID SEQUENCE OF TWO PROTEINS" JOURNAL OF MOLECULAR BIOLOGY, LONDON, GB, Bd. 48, 1970, Seiten 443-453, XP000857460 ISSN: 0022-2836**

**Beschreibung**

Die vorliegende Erfindung betrifft Verfahren zur Konformationsanalyse von Aminosäuresequenzen. Die Erfindung betrifft insbesondere Verfahren zur Validierung der Konformation gegebener aminosäurebasierter Moleküle.

Eine wichtige Aufgabe der molekularen Bioinformatik besteht in der Organisation der komplexen und großen Datenmengen der Biowissenschaften und auch in der Aufdeckung neuartiger Informationszusammenhänge im Sinne eines *data mining.* Die durch Gensequenzierung erhaltenen Informationen sind in vielen Fällen nur dann praktisch verwertbar, wenn die funktionelle Bedeutung einer bestimmten (Gen-)Sequenz aufgeklärt wird. Gensequenzen haben jedoch im Kontext der modernen Biotechnologie nur eine begrenzte Aussagekraft; den zentralen Mittelpunkt für biologische Funktionen stellen die exprimierten Proteine dar. Seit der in den vergangenen Jahren erfolgten Bereitstellung einer qualitativ hochwertigen Datenbank der humanen Genomsequenz und anderer wichtiger Genome ist es daher der nächste große Schritt der Forschung, wichtige biologische Funktionen der Bestandteile des zellulären Proteoms zu bestimmen. Hierzu kann die vorliegende Erfindung bezüglich einer Tertiärstrukturvorhersage von Proteinen anhand der Sequenz, dem Vergleich von zwei Aminosäuresequenzen durch die Erstellung eines Alignments, sowie die Validierung von vorgegebenen Proteinstrukturen, eine wichtige und wertvolle Hilfestellungen leisten.

Der Schlüssel zum wichtigen Verständnis der biologischen und funktionellen Eigenschaften von Proteinen liegt letztendlich in ihrer präzisen, eindeutigen dreidimensionalen Struktur (Konformation) begründet; die beiden Begriffe "Konformation" und "dreidimensionale Struktur" werden im Folgenden synonym verwendet. *Life Sciences*-Unternehmen benötigen solche biologischen Eigenschaften beispielsweise zur Einschätzung und Optimierung von experimentellen Arbeiten, und zum Auffinden von neuen Funktionen und Eigenschaften von Proteinen. Ein computergestütztes Verfahren zur Modellierung der Struktur kann rasch und kostengünstig erfolgen, es kann ohne Vorhandensein von (mühsam herzustellendem) Material durchgeführt werden, und es kann oftmals wesentliche Eigenschaften des untersuchten Zielproteins bereits vor der aufwendigen experimentellen Strukturaufklärung korrekt darstellen. Die Erstellung solcher Strukturmodelle ist daher ein wichtiger Teil der modernen molekularen Bioinformatik; das Faltungsproblem, also die Vorhersage der Tertiärstruktur von Proteinen aufgrund von Sequenzinformationen, gilt heute als die Königsdisziplin der Bioinformatik. Bis heute ist noch nicht verstanden, nach welchem Mechanismus sich eine gegebene Aminosäuresequenz zu einer nativen und funktionellen Protein-Tertiärstruktur faltet, somit existiert auch kein eindeutiger mathematischer Algorithmus zur Ableitung der Tertiärstruktur anhand von Sequenzinformationen. Die vorliegende Erfindung erlaubt in diesem Kontext die Erstellung einer zuverlässigen Modellstruktur von Proteinen sowie die Validierung (Abschätzung der Zuverlässigkeit) von Strukturen. Weiterhin können Proteine verändert werden durch künstlich eingeführte Punktmutationen, die die Eigenschaften der Proteine beeinflussen sollen; die Auswahl solcher Punktmutationen kann auf Basis der vorliegenden Erfindung rationaler als mit bisherigen Kriterien durchgeführt werden.

Bei der Vorhersage der Tertiärstruktur von Proteinen werden meist wissensbasierte Ansätze zugrunde gelegt, die derzeit als die zuverlässigsten Verfahren zur Strukturprognose angesehen werden (Böhm, Biophys. Chem. 59, 1-32, 1996). Hierbei wird versucht, bei Kenntnis der Sequenz eines unbekannten Proteins und einer dazu "verwandten" Templatstruktur durch vergleichende Modellierung (*homology modeling*) auf ein Tertiärstrukturmodell zu schließen. Eine bislang unbekannte Faltungstopologie kann mit diesem Verfahren nicht vorhergesagt werden; es wird jedoch erwartet, dass etwa bis zum Jahr 2010 im Rahmen der Initiative *"structural genomics"* alle relevanten natürlichen Topologien bekannt sein werden. Völlig neuartige Proteintopologien sind danach nur noch selten zu erwarten (Berman et al., Nature Struct. Biol. 7, 957-959, 2000). Die üblicherweise eingesetzten Verfahren der vergleichenden Modellierung sind ab einem bestimmten Verwandtschaftsgrad - etwa 50 % Sequenzidentität von unbekanntem Protein und Templat (Hilbert et al., Proteins: Struct. Funct. Genet. 7, 138-151, 1993) - relativ robust und zuverlässig, können aber auch dann Details wie beispielsweise Unterschiede in der Elektrodynamik im aktiven Zentrum eines Proteins nur mit begrenzter Auflösung darstellen. Es ist daher sehr wichtig, zu jedem Tertiärstrukturmodell auch dessen Zuverlässigkeit zu bestimmen, damit eine Überinterpretation der Modelle ausgeschlossen wird. Für die vergleichende Modellierung stehen heute verschiedene kommerzielle und nichtkommerzielle Verfahren und Algorithmen zur Verfügung.

Die Modellierung lässt sich grundsätzlich nach folgenden Arbeitsschritten durchführen:

- Identifizierung verwandter Proteine durch Vergleich auf Sequenzbasis (Sequenzhomologien) oder mit anderen Verfahren (z. B. *threading*)
- Alignment der Sequenzen von unbekanntem Protein und Elterstrukturen; es sollten möglichst viele (verschiedene) Elterstrukturen einer gemeinsamen Faltungstopologie mit eingebunden werden. Dieses Alignment ist der kritische Schritt bei der Modellierung und wird durch die vorliegende Erfindung unterstützt
- Identifizierung strukturell konservierter und variabler Regionen (Proteinkern und *loops*)
- Ableitung der Koordinaten des Proteinkerns (strukturell konservierte Bereiche, insbesondere in den Regionen periodischer Sekundärstrukturbereiche) durch bekannte Verfahren und Methoden
- Vorhersage der Konformation der *loops* (strukturell variable Bereiche) einschließlich der Modellierung von Insertionen und Deletionen in diesen Segmenten; auch hierfür sind bereits eine Vielzahl von technischen Verfahren etabliert

- Validierung der Modellstruktur und Qualitätsanalyse, ggf. noch geometrische Verfeinerung der Modellstruktur. Dieser finale Schritt wird wiederum durch die vorliegende Erfindung unterstützt.

Die Kenntnis der räumlichen Struktur von Proteinen ist die essentielle Voraussetzung für das Erkennen von zellbiologischen Zusammenhängen und Funktionen, von Regulationsmechanismen und enzymatischer Katalyse, der Interpretation von *in-vitro*-Experimenten und dem rationalen Design von Antibiotika, Vakzinen, und anderen Wirkstoffen in der molekularen Medizin. Limitierender Schritt für die experimentelle Bestimmung auf der Grundlage der Röntgenstrukturanalyse ist die Kristallisation unter geeigneten Bedingungen, um hochauflösende Strukturdaten zu erhalten; im Falle mehrdimensionaler NMR ist die molekulare Größe der auflösbaren Struktur limitiert, wenngleich sich diese Grenze ständig nach oben verschiebt. In jedem Falle ist der Materialbedarf für die experimentellen Strukturaufklärungen enorm groß, und ein Erfolg ist oftmals nicht garantiert. Die Sequenz von Proteinen (basierend auf Gensequenzen) kann hingegen in der Regel schnell und mit verhältnismäßig geringem Aufwand bestimmt werden. Die Anzahl der verfügbaren Gen- und Proteinsequenzen steigt daher heute überproportional schnell gegenüber der Zahl bekannter Strukturen.

Ein weiterer signifikanter wissenschaftlicher Fortschritt, vornehmlich im medizinischen Bereich und im Bereich der zellbiologischen Forschung, setzt eine möglichst effektive Lösung des Faltungsproblems voraus. Daneben könnten mit verlässlichen Werkzeugen zur Strukturrechnung an biologischen Makromolekülen beispielsweise auch die Zahl der essentiellen Tierversuche zur Entwicklung neuartiger Pharmaka drastisch gesenkt werden, indem potentielle Medikamente per Computerprogramm bezüglich ihrer biologischen Wirkung simuliert werden.

Eine der ursprünglichsten Bemühungen im Bereich der molekularen Strukturrechnung ist die Prognose der Sekundärstrukturen aufgrund der Aminosäuresequenz. Dabei steht die Idee im Vordergrund, dass nach korrekter Sekundärstrukturprognose ein Falten der Sekundärstrukturen zu einem dreidimensionalen Modell kombinatorisch sehr viel einfacher ist als die *ab initio-Prognose* der Struktur. Ein aktueller Vergleich der verschiedenen, derzeit publizierten Methoden zur Sekundärstrukturprognose findet sich in der Arbeit von McGuffin & Jones, Proteins: Struct.Funct.Genet. Vol. 52, S. 166-175, 2003.

Die ursprüngliche Methode nach Chou und Fasman basiert auf der Häufigkeitsverteilung von Aminosäuren in Sekundärstrukturelementen bei bekannten Proteinstrukturen. Nach der Festlegung von Konformationsparametern für alle 20 natürlichen Aminosäuren wird in der Zielsequenz nach Nukleationszentren von vier bis sechs Aminosäuren Länge gesucht, die als Initiatoren für den jeweiligen Sekundärstrukturtyp auftreten können. Die Chou/Fasman-Methode gilt mit einer mittleren Prognostizierbarkeit von 57 % als wenig erfolgreiche Methode; die Betrachtung einzelner Aminosäuren kann offenbar nicht als prädiktives Maß herangezogen werden. Im Gegensatz dazu verwendet die vorliegende Erfindung Informationen aus Oligopeptiden, das sind kurze Aminosäureabschnitte von zwei oder mehr Aminosäuren Länge. Die GOR-Methode (nach Garnier, Osguthorpe und Robson, den Autoren der Erstpublikation; Garnier et al., J. Mol. Biol. Vol. 120, S. 97-120, 1978) erweitert die Chou/Fasman-Methode um eine logarithmische Informationsfunktion für jeden Sekundärstrukturtyp. Als wahrscheinlichste Konformation wird diejenige gewählt, die die größte Differenz zwischen dieser und den beiden anderen Sekundärstrukturtypen aufweist. Hier ist mit einer mittleren Prognostizierbarkeit von 63 % zu rechnen.

Die angegebenen Methoden können durch Berücksichtigung von Sequenzhomologien zu bekannten Strukturen verbessert werden; die mittlere Prognostizierbarkeit liegt dann bei etwa 88 %. Neuronale Netze erreichen 64 % mittlere Prognostizierbarkeit bei einfacher Anwendung und ebenfalls etwa 88 % korrekte Prognostizierbarkeit, wenn homologe Sequenzen mit berücksichtigt werden. Andere Methoden versuchen ein Zusammenführen der verschiedenen Verfahren als *"joint prediction"*, um die Genauigkeit zu verbessern. Aus der bis heute noch limitierten Prognostizierbarkeit wurde abgeleitet, dass die nicht vorhersagbaren Fernwechselwirkungen zu etwa 20 bis 30 % die lokale Konformation bestimmen (Kabsch & Sander, Biopolymers Vol. 22, S. 2577-2637, 1983); dies wurde später jedoch relativiert, indem gezeigt wurde, dass primär die limitierte Größe der zugrundegelegten Protein-Datenbank für die Grenze der Methoden verantwortlich ist (Rooman & Wodak, Nature Vol. 335, S. 45-49, 1988). Insgesamt ist die Sekundärstrukturvorhersage auf Basis der heute bekannten Verfahren grundsätzlich limitiert und oftmals unzuverlässig. Die vorliegende Erfindung geht daher über die reine Sekundärstrukturprognose hinaus.

Die molekulare Energie eines Systems, d. h. die Summe aller Wechselwirkungen von Protein und umgebendem Lösungsmittel, bestimmt nach allgemein akzeptierter Ansicht die Ausbildung einer langfristig stabilen Struktur, den nativen Zustand (Anfinsen & Scheraga, Adv. Protein Chem. Bd. 29, S. 205-300, 1975; Jaenicke, Prog. Biophys. Mol. Biol. Vol. 49, S. 117-237, 1987). Dieser abstrakte Begriff ist zunächst darstellbar als thermodynamisches Ensemble von Konformationen, die (wie bei allen dynamischen Systemen) um eine Referenzkonformation fluktuieren. Eine Tertiärstrukturbestimmung kann in der Regel als erfolgreich angesehen werden, wenn die Referenzkonformation korrekt bestimmt wird; *"substates"* und die dynamisch erzeugten Ensembles, die für die Funktion wichtig sein können (z. B. zur Stabilisierung von Intermediaten und Übergangszuständen), sollen an dieser Stelle nicht weiter berücksichtigt werden.

Die Suche nach dem sogenannten Faltungscode (Jaenicke, Naturwissenschaften, Vol. 75, S. 604-610, 1988) wäre somit in erster Linie ein Problem der Bereitstellung einer vollständigen Energiefunktion für das komplette System, die eine präzise Unterscheidung des nativen Zustands und aller anderen, unter Nativ-Bedingungen nicht favorisierten (denaturierten) Zustände erlaubt. Gelegentlich wird angeführt, dass eine empirische Suche nach der nativen Konformation wegen der extrem großen Anzahl nicht-nativer Konformationen mit Hilfe von Computern nicht realisierbar ist, da selbst die schnellsten derzeit verfügbaren Rechner mit einer derartigen Suche bei weitem überfordert sind. Dies soll zunächst als technisches Problem an dieser Stelle nicht weiter betrachtet werden. Die zuvor beschriebene Energiefunktion ist derzeit nicht verfügbar. Die derzeitigen Versuche zur *ab initio-* Tertiärstrukturprognose folgen oft dem nachfolgenden generellen Schema (Hardin et al., Curr. Opin. Struct. Biol., Vol. 12, S. 176-181, 2002):

• Bestimmung der zu erwartenden Sekundärstruktur anhand von üblichen Methoden zur Sekundärstrukturprognose (s.o. McGuffin & Jones);
• Bestimmung einer vorläufigen Tertiärstruktur durch Berechnung einer optimalen Packung der Sekundärstrukturelemente;
• Verfeinerung der Struktur durch Anwendung von empirischen Potentialfunktionen und/oder Methoden, die auf Mustererkennung oder analogen Verfahren beruhen.

Bislang ist in der Literatur kein Ergebnis publiziert worden, das einen erfolgversprechenden Weg für die *ab-initio*-Tertiärstrukturprognose aufzeigen kann. Es muss auch bezweifelt werden, ob diese Methode einen prinzipiell praktikablen Weg für Strukturprognosen darstellt, selbst unter dem Aspekt, dass regelmäßig nur eine limitierte Korrektheit der Struktur erwartet wird. Die geforderte Hierarchie von zunächst unabhängig faltenden Sekundärstrukturelementen, die sich anschließend zusammenlagern, kann nur in beschränktem Maße auf experimentelle Befunde zurückgeführt werden.

Aufgrund des zuvor dargestellten Unvermögens, eine korrekte *ab-initio*-Tertiärstrukturprognose zu erstellen, stehen wissensbasierte Methoden zur Strukturvorhersage derzeit im Zentrum der Diskussion. Voraussetzung hierfür ist die Verfügbarkeit einer Elterstruktur, das heißt, die Struktur eines homologen Proteins (ggf. auch gleicher Funktion) und mit vermutetem evolutionären Zusammenhang zu dem untersuchten Protein. Daneben wird selbstverständlich auch die Sequenz des zu modellierenden Proteins benötigt. Die Methode ist in vielen Fällen bereits erfolgreich angewandt worden, um zumindest Modellvorstellungen über strukturelle und funktionelle Eigenschaften neuer Proteine zu gewinnen, die in der Folge Anregungen für experimentelle Untersuchungen vermitteln. Der technische Vorgang der Modellierung ist dabei heute ein weitgehend gelöstes technisches Problem; kritisch für die Modellierung ist aber zunächst ein grundsätzlich korrektes Alignment der beiden Informationen (unbekanntes Protein und Templatstruktur(en)) sowie die nachfolgende Bewertung der resultierenden Modellstruktur bezüglich deren Korrektheit. Beide wesentlichen Aspekte der Strukturprognose von Proteinen mittels vergleichender Modellierung werden durch die vorliegende Erfindung unterstützt.

Der primäre Schritt als Einstieg zur vergleichenden Modellierung ist (neben . entsprechender Literaturarbeit über bekannte experimentelle Untersuchungen zu Struktur, Funktion und Wirkmechanismen) die Bestimmung einer zuverlässigen Sequenz. Jeder Fehler in der Bestimmung der Primärstruktur hat Folgefehler bezüglich der Struktur zur Konsequenz, die im besten Falle nur lokale Wechselwirkungen tangieren, im schlimmsten Falle aber ein (lokal) falsches Alignment und damit regelmäßig ein unbrauchbares Modell verursachen. Es wird geschätzt, dass bis zu 20 % der in den Datenbanken vorhandenen Protein- und Gensequenzen zumindest partiell fehlerhaft sind.

Im nächsten Schritt wird ein Alignment erstellt, das wenigstens die zu modellierende Sequenz und das Elterprotein umfasst. Für die Erstellung von Alignments existieren verschiedene Standard-Methoden; neben paarweisem Alignment (2 Sequenzen) existieren auch Algorithmen für multiples Alignment (mit mehr als 2 Sequenzen). Das Alignment ist eine optimale (paarweise) Zusammenführung von Aminosäurepositionen mit dem Ziel, ein Minimum an Störstellen (Austausche, Insertion oder Deletion - "InDel" - von Aminosäuren) und ein Maximum an Übereinstimmungen zu realisieren. Die Bewertung der Signifikanz von Störstellen ist dabei ein variabler Faktor, der unterschiedliche Alignments zur Folge haben kann. Die Einbindung verschiedenster Bewertungsparameter wie evolutionäre Zusammenhänge von Austauschen, hydropathische oder geometrische Eigenschaften, Degeneration des genetischen Codes, Strukturinformationen des Eltermoleküls usw. kann den Erfolg von Alignments (positiv wie negativ) beeinflussen. Das Alignment ist der kritische Schritt der Modellierung; nachfolgende Beispiele zu der Erfindung geben daher verschiedene Situationen zur Erstellung von Alignments mit Hilfe der Erfindung wieder.

Nach der Identifikation der strukturell konservierten Regionen anhand des Alignments wird der eigentliche Prozess der Modellierung durchgeführt. Hierbei werden die in der Sequenz unterschiedlichen Aminosäuren an den entsprechenden Positionen in der Elterstruktur ersetzt, wobei folgende Kriterien üblich sind: (1) Beim Austausch werden alle möglichen Bindungswinkel der ursprünglichen Aminosäure so weit als möglich beibehalten; (2) überlappende *van der Waals*-Kontakte von Atomen sind nach Möglichkeit zu vermeiden; (3) im späteren Verfeinerungsprozess mittels moleklardynamischer oder Energiefunktions-Methoden sollten konservierte Gruppen, die im Eltermolekül und im Modell an identischen Positionen vorhanden sind, fixiert werden und möglichst nur ausgetauschte und neu hinzugefügte Gruppen nachträglich räumlich verschoben werden. Diese Randbedingung ist übrigens nicht aus empirischen Untersuchungen abgeleitet, sondern entstammt der Erwartung, damit evolutionäre Prozesse simulieren zu können.

Zusätzliche Aufmerksamkeit erfordert die Modellierung von *turns* und *loop*-Regionen, die Insertionen oder Deletionen aufweisen (*per definitionem* können Insertionen und Deletionen nie innerhalb von periodischen Sekundärstrukturelementen wie Helix und Faltblatt auftreten, sondern nur an deren Grenzen oder in den verbindenden *loops*). Hier existieren verschiedene Methoden zur Neubestimmung des räumlichen Verlaufes dieser *loops:* (1) Die Datenbanksuche, bei der anhand bekannter Strukturen eine optimale Geometrie für den *loop* aus einem vorgegebenen Strukturdatensatz extrahiert wird; (2) konformationelle Suche nach *loops,* die energetisch und geometrisch optimal die erforderlichen Kriterien für *loop*-Regionen erfüllen. Die Konformationssuche kann beispielsweise mittels Monte-Carlo-Methoden oder mit Hilfe von Hochtemperatur-Molekulardynamik-Simulationen erfolgen.

Am Ende des Strukturbildungsprozesses stehen üblicherweise Verfeinerungsmethoden, die Geometrie und Energieinhalt des neuen Moleküls verbessern. Diese Methoden umfassen wiederum Protokolle zur Molekulardynamik-Simulation wie auch die Anwendung von Energie-Potentialfunktionen. Gelegentlich wird jedoch beobachtet, dass derartig verfeinerte Strukturen eine vergleichsweise falschere Konformation des Modells bewirkt, als die Ausgangsstruktur war. Daher sollten Verfeinerungsmethoden vorsichtig und kritisch angewandt werden.

Schließlich sollte noch eine Evaluierung der Zuverlässigkeit des Modells vorgenommen werden. Dafür standen bisher kaum einsatzfähige Werkzeuge zur Verfügung (Novotny et al., Proteins, Vol. 4, S. 19-30, 1988; Bowie et al. , Science, Vol. 253, S. 164-170, 1991). Eine kritische Betrachtung des Modells ist in jedem Falle erforderlich; es ist nur selten zu erwarten, dass ein Modell gegenüber experimentell ermittelten Strukturen funktionelle Aspekte bis zu atomarer Auflösung reproduzieren kann. Daher muss bei der Analyse der vom Modell implizierten neuen Ideen darauf geachtet werden, dass prinzipiell jedes Modell nur Aussagen begrenzter Auflösung zulässt. Das in dieser Erfmdung beschriebene Verfahren ist in exzellenter Weise dazu geeignet, eine Validierung bzw. Bewertung der prognostizierten Struktur durchzuführen.

Sudarsanam et al. beschreiben in "Sequence-dependent conformational sampling using a database of $\phi_{i+1}$ and $\psi_i$ angles for predicting polypeptide backbone conformations (Protein Engineering, Band 10, Nummer 10, Seiten 1155-1162, 1997) ein Verfahren zur Konformationserstellung ausgehend von einer linearen Aminosäuresequenz. Hierbei wird die Aminosäuresequenz in Tetramere aufgeteilt, eine Dimeren-Datenbank generiert und die Winkel indirekt durch das Ermitteln der putativen Hexamere erzielt.

Aus "Estimation and use of protein backbone angle probabilities", Hong Seok Kang et al. (J. Mol. Biol.(1993) 229, 448-460) ist ein Verfahren zur Konformationserstellung ausgehend von einer linearen Aminosäuresequenz bekannt. Die $\phi$-$\psi$ Winkelwahrscheinlichkeiten werden auf Grundlage einer Datenbank mit bekannten Proteinstrukturen geschätzt. Diese Wahrscheinlichkeiten werden zur Vorhersage der dreidimensionalen Struktur kurzer Polypeptide genutzt.

Der vorliegenden Erfindung liegt daher die Aufgabe zugrunde, verbesserte Verfahren zur Konformationsanalyse von Aminosäuresequenzen bereitzustellen.

Diese Aufgaben wird durch den Gegenstand von Anspruch 1 gelöst. Bevorzugte Ausführungsformen der Erfindung sind in den abhängigen Ansprüchen angegeben.

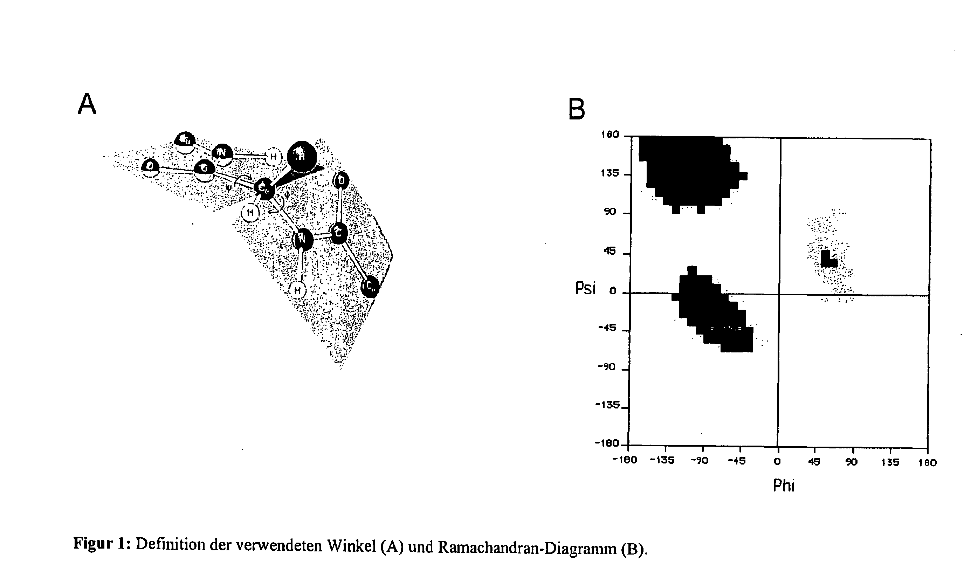

Die vorliegende Erfindung beschreibt ein Verfahren, mit dem die Validierung von vorgegebenen Strukturen durchgeführt werden kann. Dabei wird zunächst eine Datenbank von Informationen generiert, die aus kurzen Ausschnitten (Oligopeptiden) von bekannten Proteinstrukturen aufgebaut wird. Vorzugsweise, aber nicht ausschließlich, werden hierzu Tetrapeptide (vier in der Sequenz unmittelbar aufeinanderfolgende Aminosäuren) verwendet. Aus den Strukturinformationen dieser Tetrapeptide werden die Rückgratwinkel (phi/psi-Winkel; Fig. 1A) zwischen der zweiten und der dritten Aminosäure des Tetrapeptides verwendet. Diese beiden Winkel werden als typische Signatur des zugehörigen Tetrapeptides in einer Datenbank hinterlegt und hier statistisch bewertet, wie in den nachstehenden Beispielen weiter ausgeführt.

Die Erfindung betrifft ein Verfahren gemäß Anspruch 1.

Dieses Verfahren bedeutet also zunächst, dass ein aminosäurebasiertes Molekül gemäß folgendem Vorgehen in Oligopeptide gleicher Länge eingeteilt wird: wenn beispielsweise ein Molekül mit 200 Aminosäureresten in Oligopeptide mit jeweils 4 Aminosäureresten (m = 4) eingeteilt werden soll, ergibt sich die Gesamtzahl der resultierenden Oligopeptide als: 200-(4-1) = 197. Die Oligopeptide werden aus Sicht des aminosäurebasierten Moleküls in der Abfolge: 1,2,3,4; 2,3,4,5; 3,4,5,6; etc. generiert, wobei die Zahl jeweils die Aminosäureposition im aminosäurebasierten Molekül angibt.

Die Erfindung umfasst eine Einteilung in Oligopeptide, die vorzugsweise 2 - 10 Aminosäuren Länge aufweisen, wobei jedoch Tetra- und Pentapeptide bevorzugt werden. Als aminosäurebasierte Moleküle im Sinne der vorliegenden Erfindung kommen alle denkbaren aminosäurebasierten Strukturen in Betracht: Polypeptide mit einer Aminosäurelänge von ca. 10-100 Aminosäuren, Proteine mit einer Aminosäurelänge von über 100 Aminosäuren, etc. Bezüglich der Gesamtlänge der zu untersuchenden aminosäurebasierten Strukturen bestehen keine Beschränkungen. Genauso sind die erfindungsgemäßen Verfahren nicht nur auf natürliche Proteine, sondern auch beispielsweise auf durch chemische oder enzymatische Modifikation veränderte Proteine anwendbar.

So sind auch modifizierte Proteine analysierbar, die beispielsweise durch Phosphorylierung, Biotinylierung, Desamidierung, oder andere chemische Prozesse Veränderungen in der chemischen Struktur ihrer Seitenketten erfahren haben. Neben chemischen Veränderungen können Proteine auch durch die Verwendung nichtproteinogener Aminosäuren (das sind solche Aminosäuren, die nicht zum Standardrepertoire der in der Natur verwendeten 20 Aminosäuretypen gehören), beispielsweise im Rahmen einer chemischen Totalsynthese oder bei der zellfreien Herstellung von Proteinen (*in vitro*-Translation), analysiert werden.

Es sei darauf hingewiesen, dass anstelle der Wahrscheinlichkeitsdichtefunktion prinzipiell auch andere wahrscheinlichkeitstheoretische und statistische Verfahren verwendet werden können. Diese sind an sich dem Fachmann bekannte Verfahren und finden sich beispielsweise in dem Lehrbuch von Ulrich Krengel, "Einführung in die Wahrscheinlichkeitstheorie und Statistik" (7., überarb. Aufl. 2003. Vieweg Verlag, ISBN 3-528-57259-0).

In einer Ausführungsform der Erfindung wird in Schritt e) der Erwartungswert für Oligopeptide ermittelt, die gegenüber den im gegebenen aminosäurebasierten Molekül vorkommenden Oligopeptiden eine oder mehrere Aminosäuren oder Sequenzabschnitte bestimmter Länge in Form eines Ähnlichkeitsmaßstabes ausgetauscht bzw. verändert haben, wobei die Größe der Differenz zwischen beobachtetem Wert und Erwartungswert ein Maß für die durch den Austausch zu erwartende Konformationsänderung ist.

Durch dieses Verfahren ist es beispielsweise möglich, eine in dem gegebenen aminosäurebasierten Molekül an einer bestimmten Postion vorliegende Aminosäure durch eine andere zu ersetzen, wobei der Erwartungswert dann auf eine Sequenz mit der veränderten Aminosäure gerichtet ist. Als Beispiel könnte ein Austausch von Ala gegen Cys dienen. Die Größe der Differenz zwischen beobachtetem Wert und Erwartungswert gibt dann einen direkten Hinweis darauf, inwiefern der Aminosäureaustausch die Konformation des Gesamtmoleküls beeinflusst. Neben Aminosäureaustauschen kann dieses Prinzip auch zur Validierung von Insertionen und Deletionen eingesetzt werden.

Ein Verfahren zur Konformationserstellung ausgehend von einer linearen Aminosäuresequenz, umfasst die folgenden Schritte (nicht erfindungsgemäß):

a) Einteilen der Aminosäuresequenz in Oligopeptide gleicher Länge, wobei die Anzahl der Oligopeptide durch die Formel:

$$n-(m-1)$$

bestimmt ist, in der n die Aminosäureanzahl im aminosäurebasierten Molekül ist und m die Aminosäureanzahl im Oligopeptid ist;

b) Bereitstellen oder Erstellen einer Oligopeptiddatenbank, die Werte der phi- und psi-Winkel für diese Oligopeptide enthält;

c) Ermitteln der psi- und phi-Winkel jedes in a) ermittelten Oligopeptids aus den Datenbankinformationen;

d) Generieren der Konformation der Aminosäuresequenz aus den in c) für jedes Oligopeptid ermittelten psi- und phi-Winkeln.

Beispielsweise kann durch computergestützte Verfahren eine Generierung der Konformation des Moleküls erfolgen.

Vorzugsweise wird der in c) ermittelte Wert jedes psi- und phi-Winkels durch das Maximum der Wahrscheinlichkeitsdichtefunktion der psi- und phi-Winkel jedes in b) bereitgestellten phi- und psi-Winkels definiert.

Gemäß eines nicht erfindungsgemäßen Aspektes wird zum besseren Verständniss der im weiteren Teil der Beschreibung präsentierten Beispiele ein Verfahren zum Alignment zweier oder mehrerer Aminosäuresequenzen beschrieben, das die folgenden Schritte umfasst :

a) Bereitstellen eines aminosäurebasierten Moleküls mit unbekannter Konformation und einer oder mehrerer Templatsequenzen;

b) Einteilen der zwei oder mehreren Templatsequenzen und des aminosäurebasierten Moleküls mit unbekannter Konformation in Oligopeptide gleicher Länge, wobei die Anzahl der Oligopeptide durch die Formel:

$$n-(m-1)$$

bestimmt ist, in der n die Aminosäureanzahl im aminosäurebasierten Molekül ist und m die Aminosäureanzahl im Oligopeptid ist,

c) Ermitteln der psi- und phi-Winkel vorzugsweise aller in der (den) Templatsequenz(en) vorkommenden Oligopeptide;

d) Bereitstellen oder Erstellen einer Oligopeptiddatenbank, die Werte der phi- und psi-Winkel für die Oligopeptide aus b) und c) enthält;

e) Alignment der Aminosäuresequenzen basierend auf dem Vergleich der erwarteten Werte der psi- und phi-Winkel für das aminosäurebasierte Molekül mit unbekannter Konformation und den beobachteten psi- und phi-Winkeln des einen oder der mehreren Templatsequenzen.

Vorzugsweise ist der in e) verwendete Wert jedes psi- und phi-Winkels durch das Maximum der Wahrscheinlichkeitsdichtefunktion der psi- und phi-Winkel jedes in d) bereitgestellten phi- und psi-Winkels für diese Oligopeptide definiert.

Wie bereits oben angesprochen, kommen erfindungsgemäß vorzugsweise Oligopeptide zum Einsatz, die jeweils aus fünf Aminosäuren bestehen (Pentapeptide).

Hierbei werden die psi- und phi-Winkel vorzugsweise zwischen der zweiten und dritten sowie dritten und vierten Aminosäure des Pentapeptids gemessen.

Besonders bevorzugt jedoch ist die Ausführungsform, bei der die Oligopeptide jeweils aus vier Aminosäuren bestehen (Tetrapeptide). Gemäß der oben angegebenen Formel n-(m-1) ergibt sich somit die Zahl der Tetrapeptide als n-3. Hierbei werden vorzugsweise die psi- und phi-Winkel zwischen der zweiten und dritten Aminosäure des Tetrapeptids gemessen.

Erfindungsgemäß kann das oben angesprochene Validierungsverfahren insbesondere dazu verwendet werden, durch Vergleich von beobachtetem Wert und Erwartungswert das aminosäurebasierte Molekül bezüglich besonderer Eigenschaften zu bewerten.

Die von Aminosäuren in Proteinstrukturen häufig verwendeten Winkel in der phi- und der psi-Region sind im sogenannten Ramachandran-Diagramm zusammengefasst, das beispielhaft in Figur 1B gezeigt ist. Diese Information aus dem Ramachandran-Diagramm ist für eine Konformationserstellung und Konformationsanalyse zunächst noch unzureichend, da grundsätzlich alle im Ramachandran-Diagramm auftretenden ("erlaubten") Bindungswinkel zwischen zwei Aminosäuren als strukturrelevant angenommen werden können.

Die grundlegende Neuerung der vorliegenden Erfindung ist es nun, die Diederwinkel zwischen zwei konkret benannten Aminosäuren in Abhängigkeit von deren benachbarten Aminosäuren zu katalogisieren. Man erhält - z.B. bei Zugrundelegung von Oligopeptiden aus vier Aminosäuren - auf diese Weise eine große Sammlung von Tetrapeptiden ("1234"), deren räumliche Struktur mit dem psi- und phi-Winkel zwischen den mittleren Aminosäuren 2 und 3 korreliert werden kann. Die statistische Analyse der Ergebnisse erfolgt dabei mit dem an sich bekannten Verfahren der nichtparametrischen Kerndichteschätzung (Kernel Density Estimation, KDE).

Das Ziel einer Kerndichteschätzung ist, die Wahrscheinlichkeitsdichtefunktion (PDF, Probability Density Function) $f(\cdot)$ einer Zufallsvariable X zu approximieren (bei n unabhängigen Beobachtungen $x_1 \ldots x_n$; eindimensionaler Fall). Der Kerndichteschätzer $\hat{f}_h(x)$ zur Abschätzung des Dichtewertes $f(x)$ der Wahrscheinlichkeitsdichtefunktion an einem Punkt x ist definiert als:

$$\hat{f}_h(x) = \frac{1}{nh} \sum_{i=1}^{n} K\left(\frac{x_i - x}{h}\right) \qquad (1)$$

$K(\cdot)$ bezeichnet die so genannte Kernfunktion, der Parameter h wird als Bandweite bezeichnet. Es existiert eine Reihe von möglichen Kernfunktionen. Sie müssen jeweils die Eigenschaften einer Wahrscheinlichkeitsdichtefunktion erfüllen, also:

$$\int_{-\infty}^{\infty} K(x)dx = 1, \ K(x) \geq 0 \qquad (2)$$

und in der Regel sind sie um Null symmetrisch und unimodal. Für die Berechnungen der Wahrscheinlichkeitsdichtefunktionen für die vorliegende Erfindung wurde der Gauss-Kern verwendet (eindimensionaler Fall):

$$\varphi(x) = \frac{1}{\sqrt{2\Pi}} e^{-\frac{x^2}{2}} \qquad (3)$$

Multivariate Kerndichteschätzungen haben das Ziel, die Wahrscheinlichkeitsdichtefunktion $f(t) = f(t_1 \ldots t_q)$ der Zufallvariablen $T = (T_1 \ldots T_q)^T$ zu approximieren. Für den q-dimensionalen Fall ist der Kerndichteschätzer definiert als:

$$\hat{f}_h(t) = \frac{1}{n} \sum_{i=1}^{n} \frac{1}{h_1 \cdots h_q} K\left(\frac{T_{i1} - t_i}{h_1}, \cdots, \frac{T_{iq} - t_i}{h_q}\right) \quad (4)$$

Im vorliegenden Fall sind vor allem zweidimensionale Kerndichteschätzer interessant, da zwei Winkel: psi und phi (Figur 1A), betrachtet werden. Der zweidimensionale Kerndichteschätzer ergibt sich aus der Multiplikation der beiden univariaten Kernfunktionen (je eine Kernfunktion für den psi-Winkel und den phi-Winkel).

Im vorliegenden Fall ergeben sich Wahrscheinlichkeitsdichtefunktionen des Typs y = f(psi, phi), das heißt, dreidimensionale Funktionen, welche die Wahrscheinlichkeit definieren, einen bestimmten Konformations-Zustand der psi/phi-Winkel zwischen den Aminosäuren 2 und 3 bei einem gegebenen Tetrapeptid zu beobachten. Die nachfolgenden Beispiele basieren auf dieser Auswertung von Tetrapeptid-Informationen. Diese Informationen werden in Form von Wahrscheinlichkeitsdichtefunktionen für jedes einzelne Tetrapeptid berechnet. Diese Liste von Wahrscheinlichkeitsdichtefunktionen ist die Grundlage für die weiteren Beispiele.

Eine mögliche Anwendung hierfür kann beispielsweise eine Konformationsanalyse eines Proteins sein. Für eine neue (durch die in der Einleitung beschriebenen Verfahren modellierte oder experimentell bestimmte) Proteinstruktur werden für jedes Tetrapeptid die psi/phi-Winkel gemessen und der Funktionswert der Wahrscheinlichkeitsdichtefunktion für dieses Wertepaar ermittelt. Durch einen Vergleich mit dem Maximum der Wahrscheinlichkeitsdichtefunktion lässt sich ermitteln, um wieviel unwahrscheinlicher das gemessene Winkelpaar im Vergleich zum Maximum der Wahrscheinlichkeitsdichtefunktion ist. Dazu werden sowohl das Maximum der Wahrscheinlichkeitsdichtefunktion als auch der Wert der Wahrscheinlichkeitsdichtefunktion (für das beobachtete psi/phi-Wertepaar) logarithmiert und voneinander subtrahiert. Die Differenz gibt an, wie viele Zehnerpotenzen unwahrscheinlicher der beobachtete Wert im Vergleich zum Erwartungswert (Maximum der Wahrscheinlichkeitsdichtefunktion) ist; in den nachfolgenden Abbildungen ist dies als Parameter DIFFMAX aufgetragen. Es ist auf diese Weise möglich, ein Bewertungssystem zu etablieren, mit dem neue Strukturen evaluiert werden können.

Eine weitere Verwendung für Wahrscheinlichkeitsdichtefunktionen ist die Etablierung eines neuartigen Alignmentverfahrens, das "oligopeptidweise" arbeitet und nicht, wie sonst üblich, über Substitutionsmatrizen. Hierbei lassen sich grundsätzlich auch bessere Ergebnisse bezüglich eines Alignments erhalten als mit herkömmlichen Methoden. Teil der Erfindung ist daher auch die Implementierung eines Verfahrens zum Struktur-Sequenz-Alignment, das beispielsweise besser als herkömmliche (matrixbasierte) Verfahren in der Lage ist, die richtige Elterstruktur für eine Strukturmodellierung zu erkennen. Das Verfahren ist nachfolgend erläutert.

Q und T seien Sequenzen der Länge q und t, jede der Sequenzen ist definiert als lineare Folge von n Symbolen eines endlichen Alphabets B:

$$B = \begin{cases} A = Ala, & C = Cys, & D = Asp, & E = Glu, & F = Phe, \\ G = Gly, & H = His, & I = Ile, & K = Lys, & L = Leu, \\ M = Met, & N = Asn, & P = \text{Pro}, & Q = G\ln, & R = Arg, \\ S = Ser, & T = Thr, & V = Val, & W = Trp, & Y = Tyr \end{cases} \quad (5)$$

Für jede Sequenz x lässt sich ein Satz $c_4^x$ von allen sequenziell aufeinander folgenden Tetrapeptiden erstellen.

Im vorliegenden Fall gilt:

$$c_4^Q = (c_{4,1}^Q, c_{4,2}^Q, \ldots, c_{4,q-4+1}^Q) \qquad (6)$$

und

$$c_4^T = (c_{4,1}^T, c_{4,2}^T, \ldots, c_{4,t-4+1}^T) \qquad (7)$$

$PDF_4^Q$ sei der zu $c_4^q$ korrespondierende Satz von Wahrscheinlichkeitsdichtefunktionen:

$$PDF_4^Q = \left(PDF_{4,1}^Q, PDF_{4,2}^Q \cdots PDF_{4,q-4+1}^Q\right) \qquad (8)$$

$\Psi_4^T$ und $\Phi_4^T$ seien die zu $c_4^T$ gehörenden Sets von Diederwinkeln, die aus der Sequenz T errechnet wurden:

$$\Psi_4^T = \left(\Psi_{4,1}^T, \Psi_{4,2}^T \cdots \Psi_{4,t-4+1}^T\right) \quad (9)$$

$$\Phi_4^T = \left(\Phi_{4,1}^T, \Phi_{4,2}^T \cdots \Phi_{4,t-4+1}^T\right) \quad (10)$$

Das Prinzip des Alignments nach vorliegender Erfindung besteht nunmehr in der Erstellung einer Struktur-Sequenzalignment-Matrix $M_{mxn}$, mit m = q-4+1 und n = t-4+1. Das semiglobale Alignment wird gemäß dem üblichen Needleman-Wunsch-Algorithmus durchgeführt. Als Scoringfunktionen wurden die im Kontext der vorliegenden Erfindung neu entwickelten Wahrscheinlichkeitsdichtefunktionen verwendet. Es wurden affine Gapstrafen gemäß dem Gotoh-Algorithmus angewandt.

$$\sigma(q_i t_i) = \begin{cases} 0 & if\ 0 \le \sigma^\bullet(q_i t_i) < 1 \\ 1 & if\ 1 \le \sigma^\bullet(q_i t_i) < 2 \\ 2 & if\ 2 \le \sigma^\bullet(q_i t_i) < 3 \\ 3 & if\ 3 \le \sigma^\bullet(q_i t_i) < 4 \\ 4 & if\ 4 \le \sigma^\bullet(q_i t_i) < 5 \\ 5 & if\ 5 \le \sigma^\bullet(q_i t_i) < 6 \\ 6 & if\ \sigma^\bullet(q_i t_i) = 6 \end{cases} \qquad (11)$$

$$\sigma^\bullet(q_i t_i) = \log\left(\frac{\max(PDF_{4,i}^Q)}{PDF_{4,i}^Q(\phi_{4,i}^T, \psi_{4,i}^T)}\right) \quad (12)$$

mit $q_i = c_{4,i}^Q$ und $t_i = c_{4,i}^T$

Die nachfolgenden Beispiele sind durch Figuren näher dargestellt und erläutert. Bei den Darstellungen und in den Beispielen wird regelmäßig der übliche Ein-Buchstaben-Code für die Bezeichnung der einzelnen Aminosäuretypen verwendet; dieser ist international standardisiert und bedarf daher keiner näheren Erläuterung.

Figur 1 zeigt die in vorliegender Erfindung verwendete Definition der Winkel und die Winkelverteilung im Ramachandran-Diagramm. (A): schematische Darstellung einer Proteinbindung, mit Definition der beiden verwendeten Winkel phi und psi. Die Definition ist jedem geeigneten Lehrbuch der Proteinstrukturforschung zu entnehmen. (B) Ramachandran-Diagramm für die proteinogenen Aminosäuren, mit Ausnahme von Glycin. Das Diagramm ist eine übliche Darstellung von erlaubten Winkeln der beiden Parameter phi und psi.

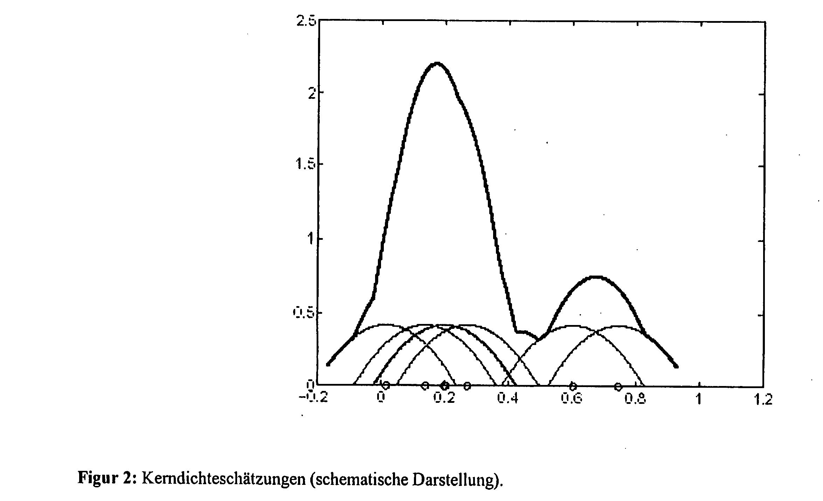

Figur 2 zeigt eine schematische Darstellung von Kerndichteschätzungen.

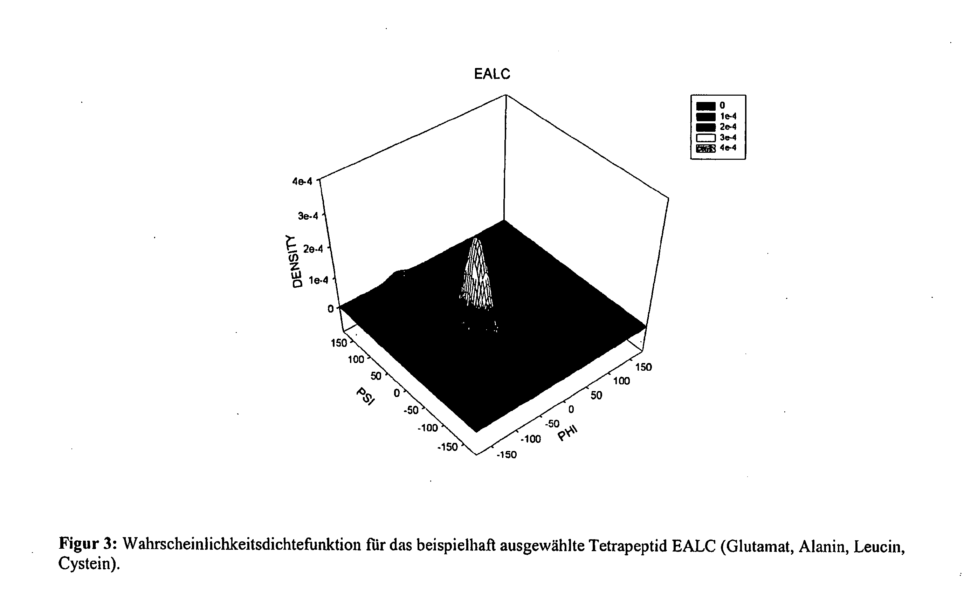

Figur 3 zeigt die Wahrscheinlichkeitsdichtefunktion für das beispielhaft ausgewählte Tetrapeptid EALC (Einbuchstabencode; dies entspricht den Aminosäuren Glutamat, Alanin, Leucin, Cystein) als Darstellung eines typischen Ergebnisses. Für die 20 proteinogenen Aminosäuren resultieren insgesamt $20^4$ = 160.000 verschiedene Wahrscheinlichkeitsdichtefunktionen, die mit Hilfe einer Analyse der in natürlichen Proteinen beobachteten Winkeln erstellt werden können. Die Analyse der vorliegenden Erfindung beruht auf den in der Protein-Datenbank (PDB; http://www.rcsb.org) veröffentlichten Proteinstrukturen.

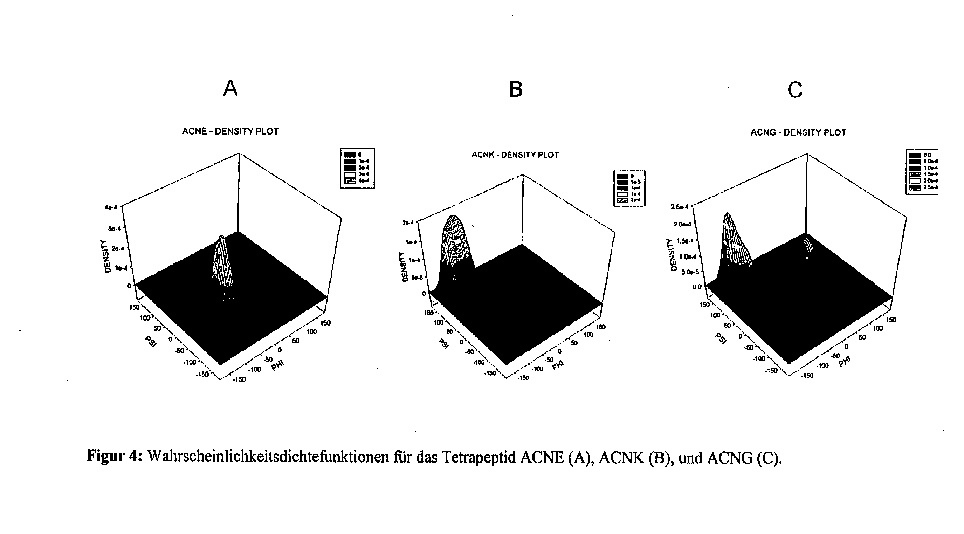

Figur 4 zeigt die Wahrscheinlichkeitsdichtefunktionen für die Tetrapeptide ACNE (A), ACNK (B), und ACNG (C). Die vierte Aminosäure des Tetrapeptids hat einen wesentlichen Einfluss auf die Dichteverteilung der phi/psi-Winkel zwischen der zweiten und dritten Aminosäure (jeweils Cystein/Asparagin). Die benachbarte Aminosäure ist als wichtiges Kriterium für die lokale Strukturbildung zu erkennen. Mit diesem Beispiel ist auch gezeigt, dass die Wahrscheinlichkeitsdichtefunktionen für die Auswahl von geeigneten Punktmutationen in Proteinen (Veränderungen einzelner Aminosäurepositionen) einen geeigneten Bewertungsmaßstab darstellen.

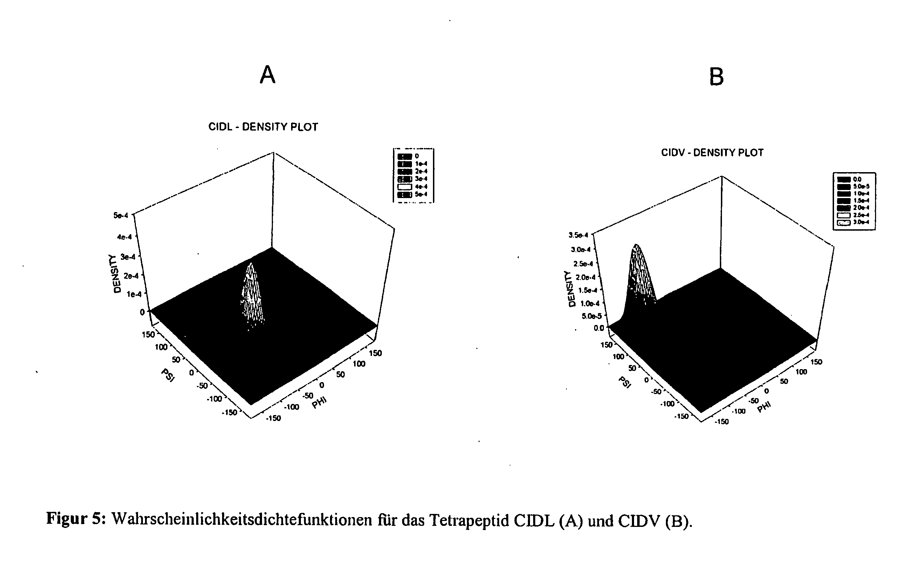

Figur 5 zeigt die Wahrscheinlichkeitsdichtefunktionen für das Tetrapeptid CIDL (A) und CIDV (B). Die vierte Aminosäure des Tetrapeptids hat wiederum einen wesentlichen Einfluss auf die Dichteverteilung der phi/psi-Winkel zwischen der zweiten und dritten Aminosäure. Gängige Substitutionsmatritzen, die für Protein Design herangezogen werden, würden bei diesem Beispiel den Austausch der beiden chemisch ähnlichen Aminosäuren Leucin und Valin (vierte Position des Tetrapeptides) favorisieren. Die Wahrscheinlichkeitsdichtefunktionen hingegen zeigen, dass bei dem Austausch der beiden hydrophoben Aminosäuren Leucin und Valin gegeneinander eine Strukturveränderung in dem Tetrapeptid zu erwarten ist. Solche Informationen sind für das rationale Protein Design besonders wichtig, wenn Punktmutationen von Proteinen geplant werden sollen; oft sollen diese Punktmutationen limitierte Funktionsänderungen bei Beibehaltung der Struktur ermöglichen. Dies ist durch die Wahrscheinlichkeitsdichtefunktionen überprüfbar.

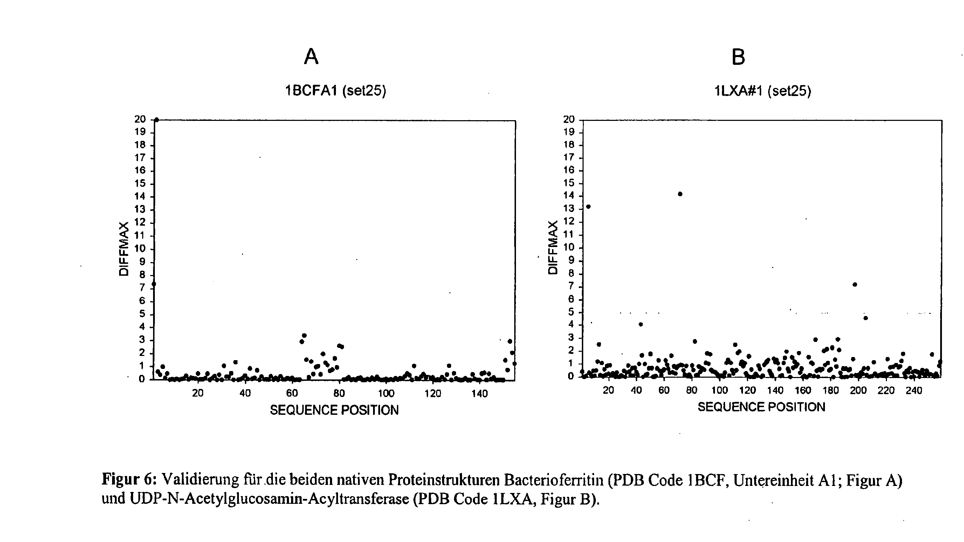

Figur 6 zeigt die Validierung für die beiden nativen Proteinstrukturen Bacterioferritin (PDB Code 1BCF, Untereinheit A1; Figur A) und UDP-N-Acetylglucosamin-Acyltransferase (PDB Code 1LXA, Figur B). Die Bewertungen durch die Wahrscheinlichkeitsdichtefunktionen zeigen, dass die beiden Strukturen als native, korrekt gefaltete Proteine erkannt werden, wie dies zu erwarten ist. Der Parameter DIFFMAX, der auf der Ordinate aufgetragen ist, gibt die Wahrscheinlichkeit wieder, dass Abweichungen von den aus den Wahrscheinlichkeitsdichtefunktionen abgeleiteten Optima wahrscheinlich toleriert werden können. Empirisch hat sich dabei eine Grenze von 5 (durchgezogene rote Linie in Fig. 6A und 6B) als Maximum der Toleranz ergeben. Geringe Abweichungen vom Optimum (Null-Linie des Graphen) sind insbesondere durch unstrukturierte Bereiche in den Proteinen zu erklären.

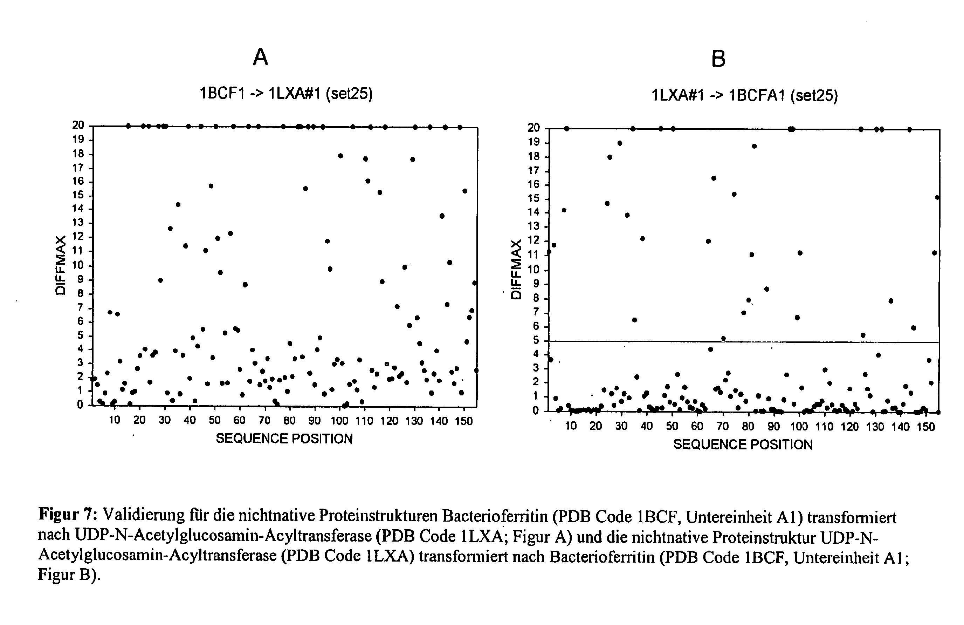

Figur 7 zeigt die Validierung für die nichtnative Proteinstrukturen Bacterioferritin (PDB Code 1BCF, Untereinheit A1) transformiert nach UDP-N-Acetylglucosamin-Acyltransferase (PDB Code 1LXA; Figur A) und die nichtnative Proteinstruktur UDP-N-Acetylglucosamin-Acyltransferase (PDB Code 1LXA) transformiert nach Bacterioferritin (PDB Code 1BCF, Untereinheit A1; Figur B). Die Bewertungen durch die Wahrscheinlichkeitsdichtefunktionen zeigen, dass die Strukturen als nicht-native bzw. inkorrekt gefaltete Proteine erkannt werden. Die DIFFMAX-Werte liegen in den beiden Proteinen ungewöhnlich oft über dem empirisch ermittelten Grenzwert von 5, das bedeutet, dass an vielen Stellen des Proteins Abweichungen von der optimalen bzw. wahrscheinlichsten Oligopeptidstruktur gefunden werden. Werte über 20 von DIFFMAX wurden in der Abbildung aus technischen Gründen auf den Maximalwert von 20 gesetzt.

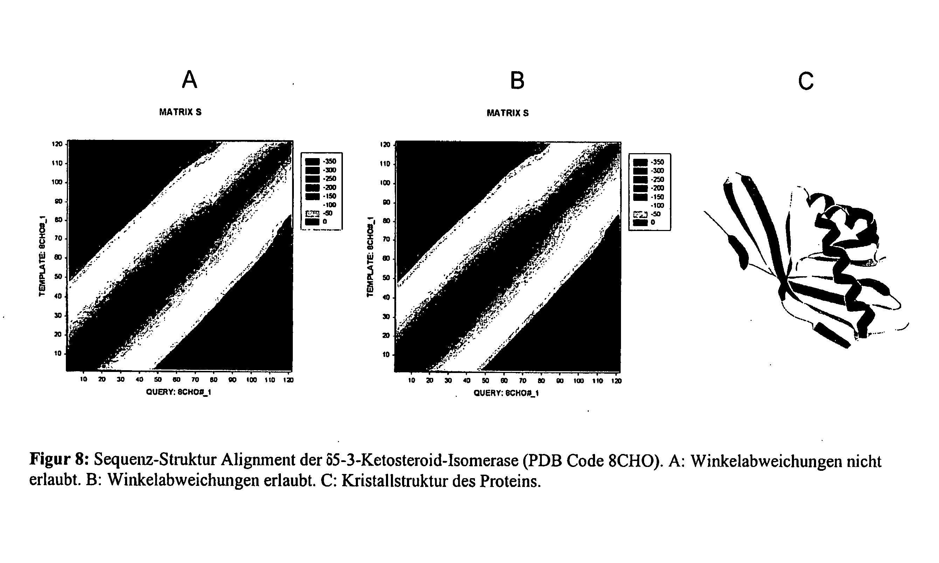

Figur 8 zeigt ein Sequenz-Struktur-Alignment mit der Sequenz von δ5-3-Ketosteroid-Isomerase gegen die eigene Proteinstruktur (PDB Code 8CHO). Die grafische Darstellung der resultierenden Matrix S zeigt die Möglichkeit auf, aus dem Alignment korrekte Information zur Auffindung eines "Pfades" zum Erstellen eines korrekten Alignments abzuleiten. Wenn in der Diagonale der Abbildung eine durchgehend rote Linie bzw. ein solcher Bereich dargestellt ist, dann gilt das Alignment als erfolgreich. Die Wahrscheinlichkeitsdichtefunktionen erlauben in Figur A keine Winkelabweichungen. Aus der Matrix R (siehe vorstehende Gleichungen) lassen sich über den Needleman-Wunsch-Algorithmus die akkumulierten Scorewerte errechnen, die anschliessend in der Matrix S berechnet und grafisch veranschaulicht werden können (A). Die Wahrscheinlichkeitsdichtefunktionen in B erlauben - im Gegensatz zur Darstellung in der Figur A - auch Winkelabweichungen (B). Die Kristallstruktur des Proteins ist in (C) dargestellt. Es ist gezeigt, dass in diesem Beispiel durch die Zulassung von Winkelabweichungen eine Verbesserung des Alignments nicht erreicht wird.

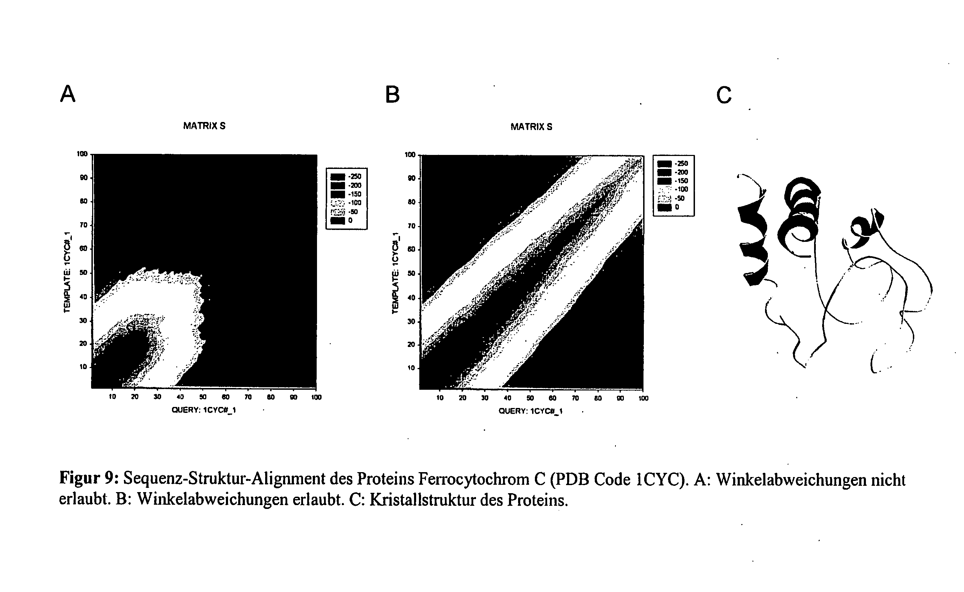

Figur 9 zeigt das Sequenz-Struktur-Alignment von Ferrocytochrom C gegen die eigene Proteinstruktur (PDB Code 1CYC). Die grafische Darstellung und Rechenmethode entsprich dem in Figur 8 gezeigten Beispiel. Die Wahrscheinlichkeitsdichtefunktionen erlauben in Figur A keine Winkelabweichungen. Aus der Matrix R lassen sich über den Needleman-Wunsch-Algorithmus die akkumulierten Scorewerte errechnen, die in der Matrix S veranschaulicht werden (A). Die Wahrscheinlichkeitsdichtefunktionen in B erlauben - im Gegensatz zur Darstellung in der Figur A - auch Winkelabweichungen (B). Die Kristallstruktur des Proteins ist in (C) dargestellt. Es ist gezeigt, dass in diesem Falle die Zulassung von Winkelabweichung in den zugrundeliegenden Wahrscheinlichkeitsdichtefunktionen ein vollständiges und korrektes Alignment erlaubt. Die Verbesserung des Verfahrens durch die Zulassung von Winkelabweichungen in vorliegendem Beispiel ist darauf zurückzuführen, dass — wie in Figur C gezeigt - das Ferrocytochrom C zu großen Teilen aus wenig strukturierten Loopbereichen besteht; periodische Sekundärstrukturelemente (Helices) sind in der Struktur nur in wenigen Bereichen realisiert.

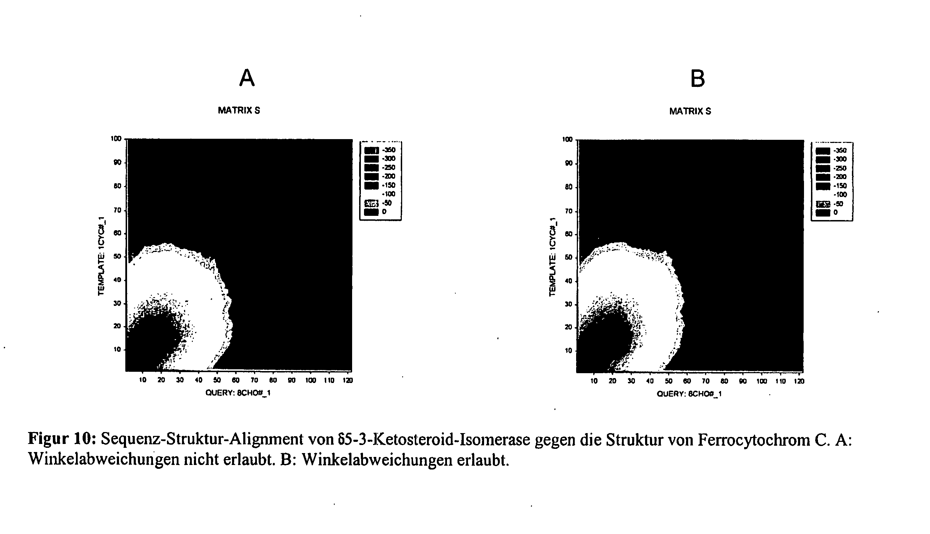

Figur 10 zeigt das Sequenz-Struktur-Alignment von $\delta$5-3-Ketosteroid-Isomerase (siehe auch Figur 8) gegen die Struktur von Ferrocytochrom C (PDB Code 1CYC; siehe auch Figur 9). Die Wahrscheinlichkeitsdichtefunktionen erlauben in Figur A keine Winkelabweichungen. Aus der Matrix R lassen sich über den Needleman-Wunsch-Algorithmus die akkumulierten Scorewerte errechnen, die in der Matrix S veranschaulicht werden (A). Die Wahrscheinlichkeitsdichtefunktionen in B erlauben - im Gegensatz zur Darstellung in der Figur A - auch Winkelabweichungen (B). Wie zu erwarten war, können die beiden nicht miteinander verwandten Sequenzen und Strukturen nicht durch ein Alignment gegeneinander abgebildet werden; dies ist unabhängig von der Zulassung von Winkelabweichungen in den zugrunde liegenden Wahrscheinlichkeitsdichtefunktionen. Damit ist gezeigt, dass Alignments (mit und ohne Zulassung von Winkelabweichungen in den Wahrscheinlichkeitsdichtefunktionen) nur dann erstellt werden können, wenn eine strukturelle Verwandtschaft der Proteine besteht; nichtverwandte Proteine werden durch das Verfahren als solche erkannt und das Alignmentverfahren zeigt deutlich die Nicht-Alignierbarkeit der beiden Proteine zueinander.

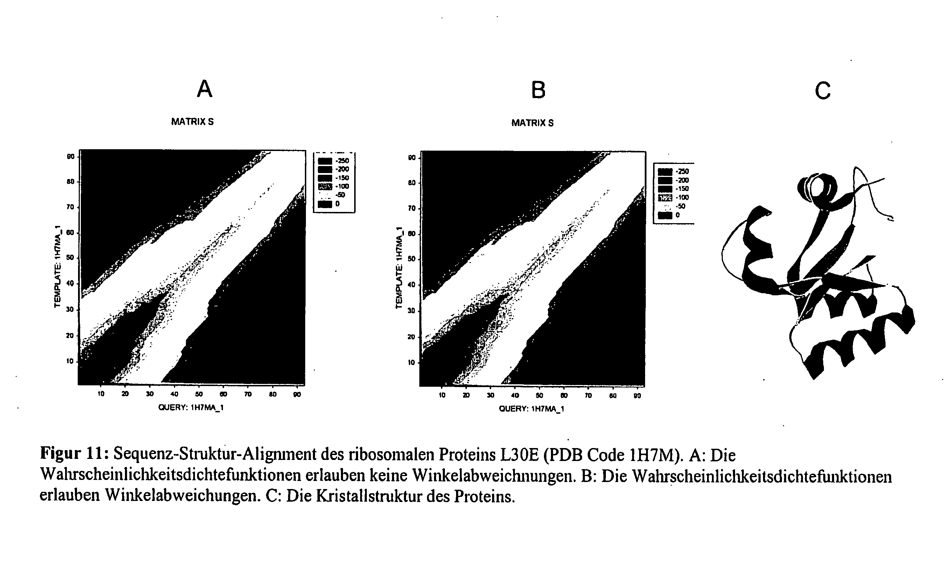

Figur 11 zeigt das Sequenz-Struktur-Alignment vom ribosomalen Protein L30E gegen die eigene Proteinstruktur (PDB Code 1H7M). Dieses Protein war eines der Zielstrukturen im fünften CASP-Wettbewerb (CASP 5, Dezember 2002). (A) Die Wahrscheinlichkeitsdichtefunktionen erlauben hier keine Winkelabweichnungen. Aus Matrix R lassen sich über den Needleman-Wunsch-Algorithmus die akkumulierten Scorewerte errechnen, die in Matrix S veranschaulicht sind. (B) Die Wahrscheinlichkeitsdichtefunktionen erlauben Winkelabweichungen. Aus Matrix R lassen sich über den Needleman-Wunsch-Algorithmus die akkumulierten Scorewerte errechnen, die in Matrix S veranschaulicht sind. Das Beispiel zeigt, dass retrospektiv eine Validierung des Verfahrens im letzten CASP-Wettbewerb grundsätzlich erfolgreich gewesen wäre. Weiterhin ist gezeigt, dass für neue Proteine, die noch nicht in der Datenbank hinterlegt sind, das Verfahren erfolgreich angewandt werden kann.

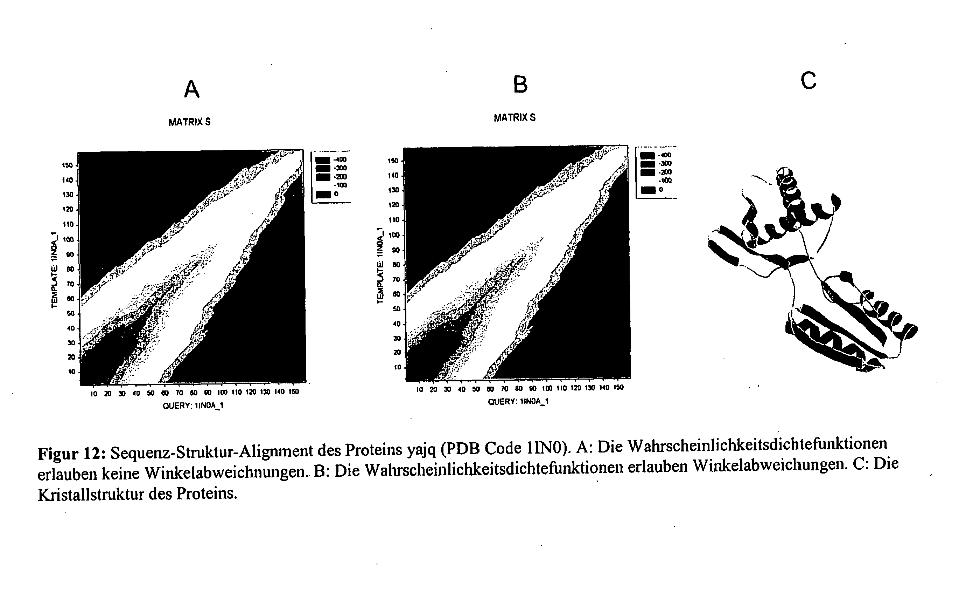

Figur 12 zeigt das Sequenz-Struktur-Alignment mit der Sequenz vom Protein yajq gegen die eigene Proteinstruktur (PDB Code 1IN0). Dieses Protein war eine der Zielstrukturen im fünften CASP-Wettbewerb (CASP 5, Dezember 2002). (A) In diesem Beispiel wurde die Datenbank ohne Winkelabweichung benutzt. Aus der Matrix R lassen sich über den Needleman-Wunsch-Algorithmus die akkumulierten Scorewerte errechnen, die in Matrix S veranschaulicht sind. (B) Die Wahrscheinlichkeitsdichtefunktionen erlauben Winkelabweichungen. Aus der Matrix R lassen sich über den Needleman-Wunsch-Algorithmus die akkumulierten Scorewerte errechnen, die in Matrix S veranschaulicht sind. Die Kristallstruktur des Proteins ist in (C) dargestellt. Das Beispiel zeigt, wie auch bereits in Figur 11 dargestellt, dass retrospektiv eine Validierung des Verfahrens im letzten CASP-Wettbewerb grundsätzlich erfolgreich gewesen wäre. Weiterhin ist wiederum gezeigt, dass für neue Proteine, die noch nicht in der Datenbank hinterlegt sind, das Verfahren erfolgreich angewandt werden kann.

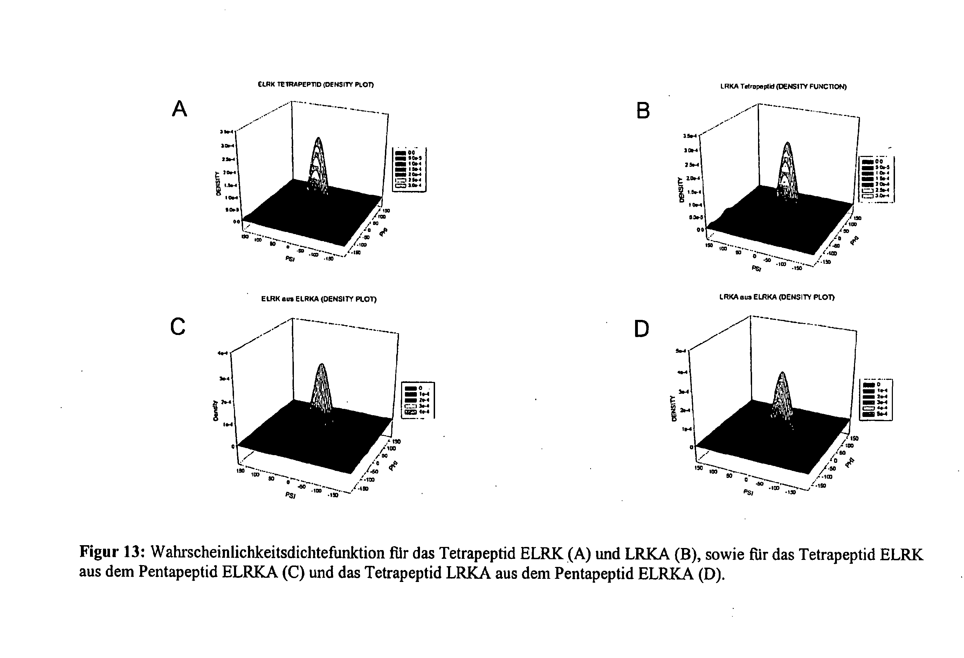

Figur 13 zeigt die Wahrscheinlichkeitsdichtefunktion für das Tetrapeptid ELRK (A) und LRKA (B), sowie für das Tetrapeptid ELRK aus dem Pentapeptid ELRKA (C) und das Tetrapeptid LRKA aus dem Pentapeptid ELRKA (D). Das Verfahren ist grundsätzlich nicht auf Tetrapeptide als zugrundeliegende Oligopeptid-Einheit beschränkt, sondern kann auch auf Basis von Oligonukleotiden anderer Länge durchgeführt werden. Durch die Verwendung von Pentapeptid-Informationen, hier durch UND-Verknüpfung entsprechender Tetrapeptidinformationen, kann eine neue und bezüglich der Winkelverteilungen im Pentapeptid auch deutlich stringentere Information gewonnen werden.

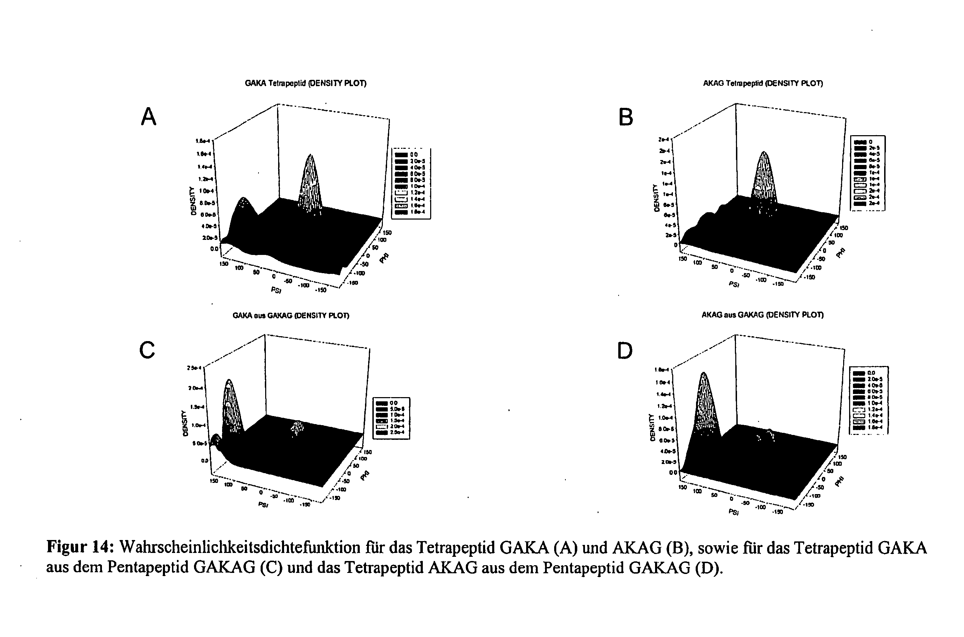

Figur 14 zeigt die Wahrscheinlichkeitsdichtefunktion für das Tetrapeptid GAKA (A) und AKAG (B), sowie für das Tetrapeptid GAKA aus dem Pentapeptid GAKAG (C) und das Tetrapeptid AKAG aus dem Pentapeptid GAKAG (D). Wie in der Figur 13 bereits dargestellt, entstehen durch die Verwendung von Oligopeptiden anderer Länge grundsätzlich vergleichbare Informationen, die aber zusätzlichen Informationsgehalt haben können.

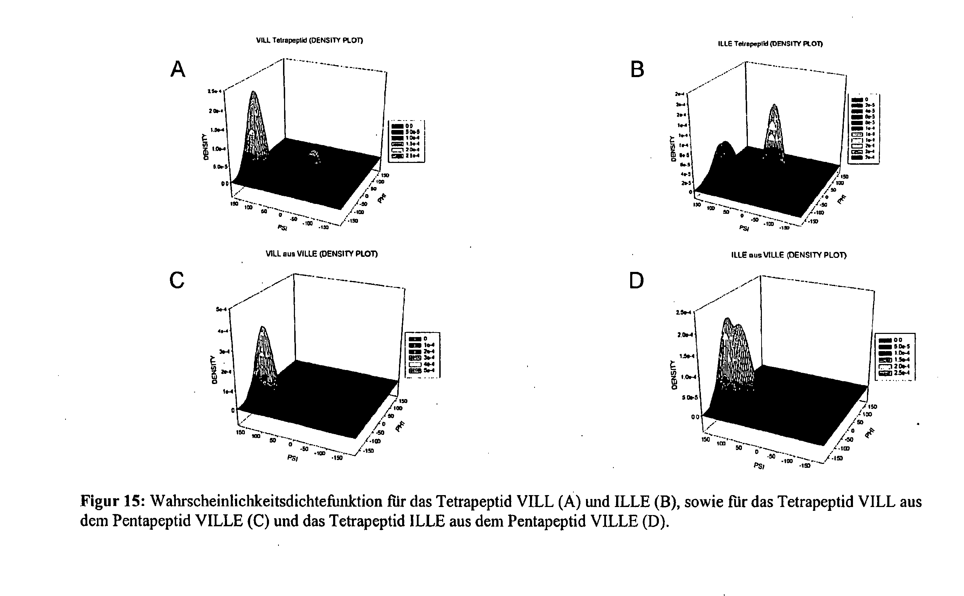

Figur 15 zeigt die Wahrscheinlichkeitsdichtefunktion für das Tetrapeptid VILL (A) und ILLE (B), sowie für das Tetrapeptid VILL aus dem Pentapeptid VILLE (C) und das Tetrapeptid ILLE aus dem Pentapeptid VILLE (D). Die Verteilungen der Winkel im Tetrapeptid bzw. im respektiven Pentapeptid zeigen interessante Korrelationen, die als zusätzliche Information bei der Strukturmodellierung und Strukturvalidierung genutzt werden können.

**Beispiel 1:**

**Aufbau einer Konformationsdatenbank: Ermittlung von Kerndichtefunktionen**

Die Anzahl der möglichen Tetrapeptide, die analysiert werden können, ergibt sich zu $20^4=160.000$ (bei 20 in der Natur beobachteten, proteinogenen Aminosäuren; Sonderfälle wie Selenocystein werden an dieser Stelle nicht gesondert betrachtet). Zur Ermittlung der statistischen Datenbasis wurden bekannte Röntgenkristallstrukturen von Proteinen tetrapeptidweise untersucht. Für eine gegebene Proteinkette aus n Aminosäuren ergeben sich somit (n-3) mögliche Tetrapeptide. Für die nachstehend beschriebenen Beispiele wurden die Diederwinkel zwischen den mittleren Aminosäuren der Tetrapeptide errechnet und tabellarisch für die spätere statistische Analyse gelistet.

Die Bestimmung der Diederwinkel setzt voraus, das für den psi-Winkel die Atome N(n)-CA(n)-C(n)-N(n+1) und für den phi-Winkel die Atome C(n)-N(n+1)-CA(n+1)-C(n+1) vollständig determiniert sind (fehlende Atome werden nicht hinzumodelliert); die Atome der beiden benachbarten Aminosäuren müssen nicht zwingend vollständig aufgelöst sein.

An die Qualität der ausgewählten, vorgegebenen Proteinstrukturen zur Berechnung der Wahrscheinlichkeitsdichtefunktionen anhand von Tetrapeptiden (s. nachstehend) wurden folgende Auswahlkriterien gestellt:

- Die Auflösung des Proteins ist besser als 3Å
- Der R-Faktor für die Strukturaufklärung beträgt 2,5 oder besser; wenn der R-Faktor unbekannt ist, dann wird dieser auf den Wert von 2,5 gesetzt.
- Die Proteinkette muss mindestens 30 Aminosäuren haben; kleinere, meist unstrukturierte Peptide werden dadurch aus der Konformationsbetrachtung ausgeschlossen.

In den heute bekannten Proteinstrukturen finden sich oft längere Bereiche, deren Struktur aus experimentellen Gründen nicht aufgelöst ist (sogenannte *gaps*). Würden jedoch die Diederwinkel zwischen zwei randständigen Aminosäuren eines *gaps,* an den Positionen N und N+m (mit m > 1) errechnet werden, dann führte dies offensichtlich zu falschen Ergebnissen. Daher war es notwendig, solche *gaps* in Proteinstrukturen sicher zu erkennen und auszuschalten. Hierzu wurde ein geometrisches Verfahren genutzt:

- Die Proteinstruktur wird primär über das Proteinrückrat (*backbone*) definiert. Die Abstände der an der Peptidbindung beteiligten Atome (N, CA, C, 0) sind aufgrund der kovalenten Natur der Bindungen weitgehend konstant. Sie liegen zwischen den beiden N-Atomen im Bereich von zwei bis fünf Å (Angström), gleiches gilt für die anderen Atome (CA/CA, C/C, O/O).
- Ausnahmen von dieser Regel findet man in *turns,* in denen die Abstände für ein bis zwei Atompaare größer sein können.
- Sollten bei mehr als zwei Atompaaren Abweichungen zu messen sein, die nicht den vorstehend genannten Kriterien entsprechen, dann wird dies als ein *gap* zwischen diesen Aminosäuren erkannt, d.h. es werden in der Folge keine Diederwinkel zwischen diesen Aminosäuren gemessen.

Die Strukturinformationen für die vorgegebenen Proteine wurden der aktuellen und allgemein zugänglichen Proteindatenbank (http://www.rcsb.org, mit Stand vom 01. März 2003) entnommen.

Eine statistische Analyse der Winkelverteilung für ein konkretes Tetrapeptid setzt zunächst die Nichtredundanz der verwendeten Daten (Proteinketten) aus der vorgegebenen, hoch redundanten Protein-Datenbank voraus. Dies ist oft notwendig, um die bevorzugte Gewichtung auf eine bestimmte Topologie zu vermeiden. Andere Arbeiten gehen bei ähnlichen Fragestellungen oft von nichtredundanten Datensätzen aus; diese nichtredundanten Datensätze werden durch Alignments der Proteinsequenzen gegeneinander bestimmt. In der vorliegenden Erfindung wurde jedoch bewusst eine nahezu vollständige (das heißt, auch teil-redundante) Proteindatenbank für die Errechnung der Diederwinkel verwendet. Man erhält hieraus für ein bestimmtes Tetrapeptid eine redundante Liste mit Proteinsequenzen, in der die Diederwinkel für dieses Tetrapeptid aufgelistet sind. Um die Redundanz aus dieser Liste anschließend (nachträglich) zu entfernen, wurden nunmehr die Proteinsequenzen gegeneinander aligniert.

Dazu wurde der Algorithmus nach Needleman-Wunsch verwendet, der zur Bestimmung optimaler, globaler Alignments zweier Sequenzen Anwendung findet (Needleman, S. B., Wunsch, C. D., J.Mol.Biol. (1970) 48:443-453). Bei unterschiedlichen Längen der Proteinsequenzen oder bei sich nur an den Enden überlappenden Alignments führen globale Alignments zu Fehlern in der Bewertung, da *gaps* am Anfang und am Ende von Proteinsequenzen bestraft werden. Derartige Probleme findet man besonders bei unterschiedlichen Sequenzlängen. Aus diesem Grund wurden semiglobale Alignments errechnet, d.h. *gaps* am Anfang und am Ende einer Sequenz wurden nicht bestraft. Die *gaps* innerhalb einer Sequenz wurden mit dem Verfahren nach Gotoh bestimmt (affine Gapstrafen, Gotoh, J. Mol. Biol. (1982) 162:705-708).

Als Substitutionsmatrix für die Alignments wurde die BLOSUM62-Matrix (Pearson, Methods Enzymol. Vol. 266, S. 227-258, 1996) gewählt. Die *open penalty* wurde auf einen Wert von "-5" und die *extension penalty* auf einen Wert von "-2" gesetzt. Für die *open penalty* entspricht dies einen um eins kleineren Wert, als der kleinste Wert in der BLOSUM62-Matrix. Damit wird verhindert, dass eine bestimmte Insertion/Deletion (zusammenfassend als InDel bezeichnet) vor einer Substitution bevorzugt wird.

Das Prinzip für die Erstellung einer nichtredundanten Liste von Tetrapeptiden mit zugehörigen Konformationen zwischen den Aminosäuren 2 und 3 des Tetrapeptids, basierend auf einem vorgegebenen Grenzwert der Ähnlichkeit der beiden Ketten (Sequenzidentität), lässt sich grundsätzlich wie folgt beschreiben:

1. Die Proteinketten werden in einer Primärliste der Länge nach sortiert.

2. Das längste Protein wird der Ergebnisliste mit den nichtredundanten Proteinen hinzugefügt (Sequenzidentität der beiden Ketten kleiner oder gleich 25%).

3. Es werden nacheinander alle kürzeren Proteinketten gegen die längste Proteinkette aligniert (gegen die Proteinkette, die in Schritt 2 der Ergebnisliste beigefügt wurde). Diejenigen Proteinketten, die eine Identität zum längsten Protein haben, die größer als der gesetzte Grenzwert ist (z.B. 25% Sequenzidentität), werden aus der Primärliste entfernt; andernfalls bleibt das betreffende Protein in der Primärliste.

4. Wenn die Primärliste vollständig abgearbeitet wurde, wird wiederum das längste Protein aus der Primärliste entnommen, der Ergebnisliste hinzugefügt und anschließend erneut Schritt 3 durchgeführt.

5. Wenn die Primärliste keine Proteinketten mehr enthält, befinden sich in der Ergebnisliste nurmehr solche Proteine, die untereinander jeweils eine Sequenzidentität haben, die kleiner ist als der gesetzte Grenzwert.

Das oben beschrieben Vorgehen erhöht den Informationsgehalt der finalen Wahrscheinlichkeitsdichtefunktionen um etwa das Vierfache, was maßgeblich zur Qualität der Wahrscheinlichkeitsdichtefunktionen beiträgt. In den nach vorliegendem Beispiel errechneten 146.300 Wahrscheinlichkeitsdichtefunktionen (146.300 beobachtete Tetrapeptide aus der Strukturdatenbank) sind Strukturinformationen aus insgesamt 12.170 Proteinketten gespeichert.

Würde dagegen eine Datenbank aus nichtredundanten Proteinstrukturen als primäre Datenbasis verwendet werden (derzeit 3.002 Proteine bei einer als Grenzwert angenommenen Sequenzidentität kleiner oder gleich 25%), wäre eine statistische Analyse für die Wahrscheinlichkeitsdichtefunktionen aufgrund des geringen Informationsgehalts der insgesamt nichtredundanten Ketten nicht mehr möglich.

Trotz der sekundären Redundanz der 12.170 Proteinketten ist somit in keiner der resultierenden Wahrscheinlichkeitsdichtefunktionen noch redundante Information enthalten. Es ist dabei zu bemerken, dass die Liste nichtredundanter Proteine (3.002) eine vollständige Untermenge der final verwendeten 12.170 Proteine ist.

Aus den Daten in der Ergebnisliste (psi/phi-Winkel für gegebene Tetrapeptide aus nichtredundanten Proteinsequenzen) werden die Wahrscheinlichkeitsdichtefunktionen errechnet. Hierzu wurde in vorliegendem Beispiel das oft verwendete Programm "R" mit der sogenannten "sm"-Bibliothek verwendet (Adrian W. Bowman und Adelchi Azzalini, "Applied Smoothig Techniques for Data Analysis", Oxford Statistical Science Series 18).

Das Prinzip der nichtparametrischen Kerndichteschätzung besteht zunächst darin, dass man versucht, eine Punktverteilung ohne funktionellen Zusammenhang mathematisch zu beschreiben (Figur 2). In diesem Verfahren legt man über jeden Punkt einer Punktverteilung eine Verteilungsfunktion (beispielsweise eine Gaußfunktion) und addiert die sich überlappenden Bereiche der Verteilungsfunktionen auf. Man erhält dadurch eine Häufigkeitsverteilung, deren einzelne Punkte die Werte der Wahrscheinlichkeitsdichtefunktion an einer bestimmten Stelle repräsentieren. Die Wahrscheinlichkeitsdichtefunktionen werden anschließend normiert, das heißt, bei zweidimensionalen Funktionen ist die Fläche unter der Kurve gleich 1, während bei dreidimensionalen Funktionen das Volumen unter der Fläche gleich 1 ist.

Beispielhaft für ein Ergebnis aus der berechneten Liste von 146.300 Tetrapeptiden ist in Figur 3 die Wahrscheinlichkeitsdichtefunktion des Tetrapeptids EALC (Angaben im Ein-Buchstaben-Code für Aminosäuren; entspricht der Sequenz Glutamat, Alanin, Leucin, Cystein) dargestellt. Die berechnete Winkelverteilung zeigt eine deutliche Präferenz für die Winkel psi = -40° und phi = -60°. Es werden in der Liste bekannter Proteinstrukturen praktisch keine anderen Winkel beobachtet, obwohl das Ramachandran-Diagramm für die genannten Aminosäuren auch andere Winkel zulässt.

**Beispiel 2**

**Analyse und Optimierung der Wahrscheinlichkeitsdichtefunktionen**

Die Diederwinkel aus Beispiel 1, die ein bestimmtes Tetrapeptid beschreiben, werden mit Hilfe der nichtparametrischen Kerndichteschätzung analysiert. Die Analysen werden dabei mit dem Softwarepaket "R" und dem zugehörigen Paket "sm" durchgeführt. Das Paket "sm" enthält dabei entsprechende Funktionen, die eine Wahrscheinlichkeitsdichtefunktionsanalyse mit dem Statistikprogramm "R" ermöglichen. Die verwendete Funktion und Parameter werden nachstehend aufgelistet:

| xlim | = cbind(-180,180) | x Achse steht für phi-Winkel; Bereich von -180 bis 180 |
|------|-------------------|---------------------------------------------------------|
| ylim | = cbind(-180,180) | y Achse steht für psi-Winkel; Bereich von -180 bis 180 |

(fortgesetzt)

| ngrid | = 91 | Das Koordinatensystem wird in 91 * 91 Zellen unterteilt; entspricht eine Skalierung der Achsen in Intervallen zu je 4 Winkelgraden |
|---|---|---|

Die Bandweite der Wahrscheinlichkeitsdichtefunktion wurden zunächst auf *"default"* eingestellt. Dabei wird die Bandweite nach Sheather-Jones ermittelt und von der Funktion intern genutzt. Es wurde jedoch festgestellt, dass eine manuell festzulegende Bandweite notwendig ist; die Standardbandweite interpoliert die Wahrscheinlichkeitsdichtefunktion zu stark. Das Ergebnis einer *default*-Berechnung ist, dass damit Winkelwahrscheinlichkeiten geschätzt werden, die nach dem Ramachandran-Diagramm nicht erlaubt wären. Es wurden daher Bandweitenanalysen durchgeführt; zunächst wurden Bandweitenwerte von 5 bis 30 in Intervallen von 5 eingesetzt und das Ergebnis der Funktionen analysiert. Als repräsentatives Tetrapeptid wurde hierbei AWQC verwendet. Unter weiterer Berücksichtigung, dass der psi-Winkel einen größeren Spielraum im Ramachandran-Diagramm hat als der phi-Winkel, wurden für die optimalen Bandweiten die Werte 15 für phi und 25 für psi ermittelt.

Anschließend wurden die Wahrscheinlichkeitsdichtefunktionen von verschiedenen Tetrapeptiden miteinander verglichen. Besonders interessant sind hierbei solche Tetrapeptid-Paare, die sich lediglich durch eine einzige Substitution unterscheiden. Nach der Substitutionsmatrix BLOSUM62 wird so beispielsweise der Austausch von Glutamat (E) gegen Lysin (K) in der Tetrapeptid-Sequenz ACNE und ACNK mit einer Bewertung von +1 bewertet, d.h. Glutamat und Lysin werden als weitgehend homologe Aminosäuren behandelt. Die Wahrscheinlichkeitsdichtefunktionen zeigen für die Tetrapeptide ACNE und ACNK aber deutlich verschiedene Winkelverteilungen. Eine einfache Substitution der beiden Aminosäuren ist hiernach nicht erlaubt. Man erkennt hierbei, dass die BLOSUM62-Matrix (wie alle gängigen Substitutionsmatrices) nur eingeschränkt für Alignments verwendet werden kann.

Beispielhaft wurden folgende Tetrapeptide verglichen: ACNE, ACNK, und ACNG; sowie CIDV und CIDL.

Figur 4 zeigt das grafische Ergebnis der Wahrscheinlichkeitsdichtefunktionen für die beispielhaft ausgewählten Tetrapeptide ACNE (Figur 4a), ACNK (Figur 4b), und ACNG. Die vier Tetrapeptide unterscheiden sich jeweils lediglich in der letzten Aminosäure, der Sequenz-Unterschied der vierten Aminosäure liegt somit zunächst außerhalb des Winkelbereiches der zweiten und dritten (betrachteten) Aminosäure. Dennoch hat die vierte Aminosäure des Tetrapeptids einen wesentlichen Einfluss auf die Dichteverteilung der phi/psi-Winkel zwischen der zweiten und dritten Aminosäure.

Anhand dieses Beispieles ist nochmals besonders hervorzuheben, dass ein Austausch von Glutamat (E) gegen Lysin (K) nach herkömmlichen Bewertungsschemata (z.B. der oft verwendeten BLOSUM62-Matrix) ausdrücklich erlaubt ist (Wert von "+1" in der Substitutionsmatrix). Wie man an den Wahrscheinlichkeitsdichtefunktionen erkennen kann, kann eine derartige Substitution jedoch lokal zu einer falschen Konformation führen, die global eine fehlerhafte Tertiärstruktur zur Folge hätte. Das Verfahren der vorliegenden Erfindung erkennt diesen Sachverhalt und würde bei einem Alignment oder bei einer Validierung diesen Fehler umgehen. Ebenfalls kann diese Information nutzbringend zur Planung von veränderten Proteinen (im Kontext von Protein Design) verwendet werden.

Durch einen Vergleich der Wahrscheinlichkeitsdichtefunktionen untereinander lassen sich Konsensussequenzen finden, die unabhängig von der Aminosäurezusammensetzung (und unabhängig von der Tertiärstruktur) immer die gleiche Struktur aufweisen. Dieser Sachverhalt kann beispielsweise bei einem *de novo* Design von Proteinen Anwendung finden, bei dem gezielt Eigenschaften (beispielsweise Bindungsaffinitäten, Löslichkeiten, Oberflächeneigenschaften) von Proteinen verändert werden können, ohne dabei die Tertiärstruktur des Rückgrats des Proteins wesentlich zu verändern.

Figur 5 zeigt das grafische Ergebnis der Wahrscheinlichkeitsdichtefunktionen für die wiederum beispielhaft ausgewählten Tetrapeptide CIDV (Figur 5a) und CIDL (Figur 5b). Die vier Tetrapeptide unterscheiden sich wiederum lediglich in der letzten Aminosäure, der Sequenz-Unterschied der vierten Aminosäure liegt außerhalb des Winkelbereiches der zweiten und dritten (betrachteten) Aminosäure. Dennoch hat die vierte Aminosäure auch in diesem angegebenen Beispiel von Tetrapeptiden einen wesentlichen Einfluss auf die Dichteverteilung der psi/phi-Winkel zwischen der zweiten und dritten Aminosäure. Obwohl beide ausgetauschten Aminosäuren Leucin bzw. Valin zur Gruppe der hydrophoben Aminosäuren gehören und solche Austausche gemeinhin als konservativ gelten, ist in speziellen, durch die Wahrscheinlichkeitsdichtefunktionen beschriebenen Fällen bei einem solchen Austausch möglicherweise eine Konformationsänderung des zugrundeliegenden Oligopeptids zu erwarten.

Die beiden vorstehenden Beispiele zeigen, dass die in dem beschriebenen Verfahren angewandte Analyse von Tetrapeptiden neue Informationen erzeugen kann, die wertvolle Informationen für die Konformationsanalyse sowie die Konformationsmodellierung von Proteinen liefern kann. Aminosäuren, die außerhalb des primär betrachteten Bereiches von zwei Aminosäuren liegen, können einen profunden und signifikanten Einfluss auf die Ausprägung der Winkel zwischen den beiden Aminosäuren haben. Diese Information kann damit direkt in ein Alignmentverfahren sowie in ein Modellierungsverfahren einfließen, das solchen Verfahren ohne diese Informationen überlegen sein dürfte. Weiterhin können die Tetrapeptid-Informationen zur Validierung von vorgegebenen Proteinstrukturen dienen; diejenigen Wahrscheinlichkeitsdichtefunktionen, die eindeutige Präferenzen haben, können zur Überprüfung der Konformation in modellierten Proteinen herangezogen werden. Dieser Anwendungsfall ist in nachfolgendem Beispiel 3 beschrieben.

**Beispiel 3**

**Validierung von Proteinstrukturen**

Die Qualität und der Nutzen der gemäß Beispiel 1 erstellten Wahrscheinlichkeitsdichtefunktionen können durch Evaluierungsstudien bewertet werden. Dazu werden Proteine verwendet, die aus der allgemein zugänglichen Protein-Datenbank (PDB; http://www.rcsb.org) stammen. Im ersten Schritt werden hierfür zwei zufällig ausgewählte, strukturell einfache Proteine betrachtet: Bacterioferritin (PDB Code 1BCF, Untereinheit A1), bestehend vorwiegend aus alpha-Helices, und UDP-N-Acetylglucosamin-Acyltransferase (PDB Code 1LXA), bestehend überwiegend aus einer beta-Helix-Struktur.

Das grundsätzliche Vorgehen bei der Evaluierung von Proteinstrukturen mittels Wahrscheinlichkeitsdichtefunktionen ist wie folgt:

- Die psi- und phi-Winkel aller im jeweiligen Protein vorkommenden Tetrapeptide werden ermittelt.
- Ausgehend von den Wahrscheinlichkeitsdichtefunktionen werden über die ermittelten psi/phi-Wertepaare die logarithmierten Wahrscheinlichkeitsdichtefunktionswerte ermittelt.
- Die Bewertung wird aus der Differenz des Maximums der Wahrscheinlichkeitsdichtefunktionen und f(psi,phi) bestimmt.
- Die Bewertungen werden aminosäure-positionsweise zu jedem Tetrapeptid in einem Diagramm aufgetragen.
- Zu beachten ist, dass in diesem Beispiel aus Gründen der besseren optischen Darstellung alle Bewertungen, die größer oder gleich 20 betragen, auf den Wert 20 gesetzt wurden; Werte > 20 sind bereits so unwahrscheinlich, dass diese Vereinfachung möglich ist.

Die Scorewerte (DIFFMAX) für beide Proteine liegen im jeweiligen Diagramm (Figur 6A und 6B) vorwiegend unmittelbar an der Null-Linie. Die in den beiden Proteinen beobachteten lokalen Konformationen stimmen somit jeweils sehr gut mit den erwarteten Werten der Wahrscheinlichkeitsdichtefunktionen für die jeweiligen Tetrapeptide gut überein. Eine Abweichung der Winkel von den erwarteten Winkeln würde sich durch eine Abweichung von der Null-Linie zeigen; da die Abweichung logarithmisch aufgetragen wurde, sind die gezeigten Abweichungen besonders signifikant.

Interessant sind die von der Null-Linie abweichenden Werte von DIFFMAX im Diagramm für das Protein Bacterioferritin (Figur 6A). Die Positionen der jeweils konformationell abweichenden Tetrapeptide im Protein wurden lokalisiert und dabei festgestellt, dass diese Abweichungen ausschließlich in den *loop*-Regionen des Proteins vorkommen. Damit ist gezeigt, dass die Wahrscheinlichkeitsdichtefunktionen die Konformation von Tetrapeptiden in definierten periodischen Sekundärstrukturen im Protein sehr gut erkennen und erfassen können. Sie zeigen jedoch in den *loop*-Regionen von natürlichen Proteinen geringe Abweichungen. Dies erscheint aber verständlich, wenn berücksichtigt wird, dass *loops* undefinierte Strukturbereiche eines Proteins sind und daher größere Konformationsfreiheiten haben als die periodischen Sekundärstrukturelemente.

Kurze, sequenzidentische Segmente können in verschiedenen Proteinen unterschiedliche Strukturen aufweisen. Dieser Sachverhalt könnte zu dem Schluss führen, dass eine Bewertung der Gesamtstruktur mit Hilfe von Wahrscheinlichkeitsdichtefunktionen, die durch Oligopeptide definiert sind, nicht möglich wäre. Die vorliegende Erfindung betrachtet hingegen die geometrischen Eigenschaften eines Tetrapeptid nicht isoliert, sondern im Kontext zu den benachbarten Tetrapeptiden. Ein Tetrapeptid, das sowohl eine helikale als auch eine Faltblattkonformation annehmen kann, wird innerhalb einer Helix die entsprechende Helixkonformation annehmen, auch wenn die Faltblattkonformation theoretisch erlaubt wäre. Die errechneten Wahrscheinlichkeitsdichtefunktionen sind in der Lage, diesen Sachverhalt mit hoher Zuverlässigkeit zu erkennen und umzusetzen. Dies widerspricht auch nicht den früheren Erkenntnissen von Kabsch & Sander (Proc. Natl. Acad. Sci. U.S.A. Vol. 81, S. 1075-1078, 1984), die Pentapeptide gleicher Sequenz in verschiedenen Konformationen in Proteinen gefunden haben. Solche nichteindeutige Zuordnungen existieren tatsächlich; im Kontext einer vollständigen Strukturbetrachtung sind aber eindeutige Zuordnungen in so großer Zahl vorhanden, dass insgesamt eine statistisch valide Aussage über wahrscheinliche Konformationen in Proteine wiederum möglich wird.

Im Weiteren wurde ein künstlicher Datensatz generiert, der Proteine mit einer klar falschen Faltung enthält, und diese klar falschen Strukturen mit den Wahrscheinlichkeitsdichtefunktionen analysiert. Als Ergebnis sind geringe Wahrscheinlichkeiten für das Auftreten der entsprechenden Tetrapeptid-Konformationen in den falsch gefalteten Proteinen zu erwarten.

Die Simulation eines falsch gefalteten Proteins erfolgte — gemäß der hierfür üblichen Vorgehensweise - dergestalt, dass die Koordinaten der beiden Proteine gegeneinander ausgetauscht wurden (Novotny et al., Proteins, Vol. 4, S. 19-30, 1988). Dabei wurde die Rückgrat-Struktur der A1-Untereinheit des Proteins Bacterioferritin (PDB Code 1BCF) zu derjenigen von UDP-N-Acetylglucosamin-Acyltransferase (PDB Code 1LXA) transformiert, und umgekehrt, bzw. die Sequenzen auf das jeweils andere Rückgrat aufmodelliert. Durch diese Transformation entstehen jeweils missgefaltete Proteine, deren Faltungstopologie nicht mit der entsprechenden Sequenz kompatibel ist. Figur 7A und 7B veranschaulichen das Resultat der transformierten Proteine. Die Auswertung der entsprechenden Wahrscheinlichkeitsdichtefunktionen zeigt, dass die aus den entsprechenden Tetrapeptiden abgeleiteten präferierten Winkel in den Strukturen überwiegend NICHT realisiert werden; die in den Strukturen realisierten Winkel zeigen deutliche Abweichungen von den Maxima der Wahrscheinlichkeitsdichtefunktionen (DIFFMAX) bezüglich der Tetrapeptide. Die veränderten Proteinstrukturen zeigen im Vergleich zu den Originalproteinen (Figur 6A und 6B) an vielen Stellen sehr ungünstige Bewertungen (unwahrscheinliche Konformationen). Damit ist gezeigt, dass die berechneten Wahrscheinlichkeitsdichtefunktionen sehr gut dazu geeignet sind, um korrekt gefaltete Proteine (Abb. 6A und 6B) von falsch gefalteten Proteinen (Abb. 7A und 7B) im Ganzen oder in Substrukturen (Teilbereichen) zu unterscheiden. Die Erfindung ist damit zur Validierung einer Proteinstruktur sehr gut geeignet.

**Beispiel 4**

**Analyse mit Wahrscheinlichkeitsdichtefunktionen ohne Berücksichtigung von Winkelabweichungen**

Die Durchführung der Analyse nach Beispiel 3 für das Protein humanes Serumalbumin (PDB Code 1AO6), Untereinheit A1, zeigt bei einer Analyse der nativen Struktur bereits unerwartet hohe Abweichungen (große Differenzen zum Maximum der Wahrscheinlichkeitsdichtefunktionen). Das würde bedeuten, dass nach Interpretation gemäß Beispiel 3 das native Protein eine fehlerhafte Struktur aufweist. Aus diesem Grund wurde bei den Alignments zusätzlich zur maximalen Sequenzidentität von 25 % eine Winkelabweichung zugelassen. Dies bedeutet, dass bei einer Winkelabweichung von > 25° zwischen zwei Tetrapeptiden dieses Protein in der Wahrscheinlichkeitsdichtefunktion mit berücksichtigt wird, auch wenn es eine höhere Sequenzidentität (> 25 %) zu den anderen Proteinen aufweist. Es zeigt sich, dass dieses Vorgehen zu einer deutlichen Verbesserung der Strukturevaluierung von humanem Serumalbumin (PDB Code 1AO6) führte, ohne dass parallel eine Verschlechterung der Qualität der Wahrscheinlichkeitsdichtefunktionen beobachtet wurde. Zur Darstellung dieses Verhaltens der Wahrscheinlichkeitsdichtefunktionen wurde das vorliegende Beispiel 4 gewählt.

Aus der nichtredundanten Proteindatenbank (3002 Proteinketten, ≤ 25 % Sequenzidentität der Ketten zueinander) wurden Testdatensätze generiert. Dazu wurden die PDB-Identifizierungscodes, die alphabetisch geordnet waren, zufällig angeordnet (Zufallszahlen nach http://www.random.org). Aus dieser randomisierten Liste wurden wiederum zufällig zehn Proteine gewählt (Zufallszahlen nach http://www.random.org).

Aus den Wahrscheinlichkeitsdichtefunktionen, welche diejenigen Tetrapeptide beschreiben, aus denen die Proteinketten aufgebaut sind, wurden die Informationen (d.h. die Diederwinkel) über die vorstehend ausgewählten Proteine gelöscht und die Wahrscheinlichkeitsdichtefunktionen erneut berechnet (*jackknifing* Test). Auf diese Weise können die Wahrscheinlichkeitsdichtefunktionen an simuliert "neuartigen" Proteinstrukturen getestet werden.

Die Ergebnisse zeigen, dass es mit dem vorstehend beschriebenen Verfahren möglich ist, unbekannte Strukturen zu evaluieren. Unsichere Aussagen gibt es lediglich in einigen wenigen unstrukturierten Regionen; hier treten Abweichungen von den Erwartungswerten der Wahrscheinlichkeitsdichtefunktionen auf. Dies ist jedoch zu erwarten. Auch an den Enden von periodischen Sekundärstrukturen werden gelegentlich Fehler gefunden; dies ist darin begründet, dass die Konformation der Peptidkette an dieser Stelle aus sterischen Gründen oft eine eher ungewöhnliche Konformation einnehmen muss.

Der Erfolg neuer, zuverlässiger und automatisierbarer Algorithmen zur Tertiärstrukturvorhersage wird in einem internationalen und öffentlichen Wettbewerb bewertet (Moult et al., Proteins: Struct. Funct. Genet. Suppl 3, 2-6, 1999). Bei diesem CASP-Wettbewerb (Critical Assessment of Techniques for Protein Structure Prediction, http://predictioncenter.llnl.gov/) können Forschergruppen ihre Vorschläge für bislang unbekannte Proteinstrukturen einreichen, deren experimentelle Darstellung jeweils kurz vor der Aufklärung steht. Nach erfolgreicher experimenteller Strukturaufklärung werden die bis dato eingereichten Modelle mit der realen Struktur verglichen; dadurch werden erfolgreiche Verfahren objektiv bewertet. Der CASP-Wettbewerb ist heute anerkannter Standard bei der Beurteilung neuer Modellierungsverfahren. Für die Analyse in diesem Beispiel wurden auch zwei Proteine verwendet, die im CASP-Wettbewerb des Jahres 2002 zur Prognostizierung ausgewählt wurden.

**Beispiel 5**

**Erstellung von Alignments**

Um die Brauchbarkeit der Wahrscheinlichkeitsdichtefunktionen als neuartige Scoringfunktionen zur Anwendung auf Alignments zu überprüfen (vgl. Gleichungen 5 bis 12), wurde δ5-3-Ketosteroid-Isomerase (PDB Code 8CHO) gegen die eigene Struktur aligniert. Das Ergebnis ist in Figur 8 dargestellt. In diesem Beispiel wurde eine *open penalty* von 7 und eine *extension penalty* von 2 eingesetzt. Gleichung 12 wird zur Berechnung einer Matrix R verwendet, die dann in Figur 8A in die akkumulierten Wahrscheinlichkeitsdichtefunktionswerte (ohne Winkelabweichung), die sich entsprechend dem Gotoh-Algorithmus ergeben, umgerechnet werden. Man erkennt anhand der niedrigen akkumulierten Wahrscheinlichkeitsdichtefunktionswerte (rote diagonale Linie), dass die Struktur sehr gut erkannt wird. Figur 8B zeigt das gleiche Alignment von δ5-3-Ketosteroid Isomerase mit Wahrscheinlichkeitsdichtefunktionen, die eine Winkelabweichung zulassen. Man erkennt keine Verbesserung des Alignments gegenüber der Bewertung ohne die Zulassung von Winkelabweichungen. Dies ist darauf zurückzuführen, dass dieses Protein eine gut definierte Struktur aufweist.

Figur 9 zeigt das Sequenz-Strukturalignment von Ferrocytochrom C (PDB Code 1CYC) gegen die eigene Struktur (Wahrscheinlichkeitsdichtefunktionen ohne Zulassung von Winkelabweichungen). Aus Figur 9A ist erkennbar, dass die Struktur nur sehr schlecht erkannt wird. Ursache hierfür ist, dass das Protein bezüglich definierter Sekundärstrukturanteile (Helices und Faltblätter) praktisch strukturlos ist. In Figur 9B ist ein identisches Alignment dargestellt; in diesem Fall erlauben die Wahrscheinlichkeitsdichtefunktionen aber eine Winkelabweichung. Bemerkenswert ist, wie gut in diesem Fall mit den geänderten Wahrscheinlichkeitsdichtefunktionen im Alignment die richtige Struktur erkannt wird.

Um auszuschließen, dass die Wahrscheinlichkeitsdichtefunktionen mit den Winkelabweichungen an Schärfe verlieren, das heißt, auch bestimmte nicht erlaubte Konformationen zulassen, wurde δ5-3-Ketosteroid-Isomerase gegen das nicht damit verwandte Ferrocytochrom C aligniert. Man erkennt, dass sowohl ein Alignment mit Wahrscheinlichkeitsdichtefunktionen ohne Winkelabweichung (Figur 10A) als auch ein Alignment mit Wahrscheinlichkeitsdichtefunktionen, die Winkelabweichungen zulassen (Figur 10B) nicht zu einem zufriedenstellenden Alignment führen, was den Schluss zulässt, dass die beiden Sequenzen nicht strukturähnlich sind und ein Alignment zwischen den beiden Proteine nicht sinnvoll erstellt werden kann.

**Beispiel 6**

**Analyse der CASP 5-Proteine**

Im Wettbewerb CASP werden neue und bis dato unbekannte Proteine und Proteinstrukturen dazu verwendet, um die Fähigkeiten neuer Verfahren zur Modellierung von Proteinstrukturen nach einem allgemein akzeptierten und unabhängigen Verfahren zu überprüfen. Die CASP-Proteine eigenen sich daher hervorragend dazu, um retrospektiv die Wahrscheinlichkeitsdichtefunktionen auf ihre Fähigkeit hin zu testen, unbekannte Strukturen zu erkennen und zu bewerten. Figur 11A und Figur 11B zeigen die Alignments der Sequenzen (jeweils mit und ohne Zulassung von Winkelabweichungen der Wahrscheinlichkeitsdichtefunktionen) vom ribosomalen Protein L30E (PDB Code 1H7M) gegen die - nach Abschluss des Wettbewerbs veröffentlichte, aber in der für die Berechnungen zugrundeliegenden Datenbank noch nicht vorhandene - Struktur. Man erkennt, dass die den zugrunde liegenden Wahrscheinlichkeitsdichtefunktionen bis dato unbekannte Struktur gut erkannt wird und das Alignment erfolgreich ist. Gleiches gilt für die Sequenz vom Protein yajq (PDB Code 1IN0, Figuren 12A und 12B). Auch in diesem Fall wird die Struktur sehr gut erkannt.

Diese Beispiele zeigen, dass die vorliegende Erfindung sehr gut dazu geeignet ist, Sequenzen unbekannter Strukturen die richtige Faltungstopologie zuzuordnen bzw. auch Fehler in modellierten Strukturen aufzudecken. Weiterhin ist gezeigt, dass bis dato unbekannte Proteine mit dem Verfahren erfolgreich analysiert und bewertet werden können.

**Beispiel 7**

**Verwendung von Pentapeptiden zur Strukturvalidierung von Proteinen**

Bei der Verwendung von Tetrapeptiden ("1234") in den zugrunde liegenden Wahrscheinlichkeitsdichtefunktionen zeigte sich in vorhergehenden Beispielen eine Abhängigkeit der Diederwinkel psi und phi zwischen Aminosäure 2 und 3 von den benachbarten Aminosäuren 1 und 4. Durch eine Auswertung dieser Information ließen sich dreidimensionale Wahrscheinlichkeitsdichtefunktionen errechnen (Diederwinkel zweidimensional, dritte Dimension ist die Wahrscheinlichkeit). Das beschriebene Prinzip lässt sich außer mit Tetrapeptiden aber ebenso mit Oligomeren anderer Sequenzlänge anwenden, wie nachfolgend am Beispiel von Pentapeptiden gezeigt. Bei Pentapeptiden ("12345") erfolgt eine Bestimmung der Diederwinkel zwischen den Aminosäuren 2 und 3 sowie 3 und 4. Der Aufbau der erforderlichen Datenbank mit Pentapeptidstrukturen erfolgt sinngemäß nach den Angaben aus dem Beispiel 1. Die Anzahl möglicher Pentapeptide ergibt sich dabei zu $20^5 = 3.200.000$, tatsächlich können in der derzeit verfügbaren Datenbank von Proteinstrukturen derzeit nur 831.355 verschiedene Pentapeptide nachgewiesen werden.

Die resultierenden Wahrscheinlichkeitsdichtefunktionen sind fünfdimensional, mithin ist also eine normale grafische Darstellung nicht mehr möglich. Für eine Erläuterung des Prinzips lassen sich die fünfdimensionalen Wahrscheinlichkeitsdichtefunktionen zu jeweils zwei dreidimensionalen Wahrscheinlichkeitsdichtefunktionen vereinfachen (Tetrapeptid "1234" und Tetrapeptid "2345"). Eine Aussage, um wieviel unwahrscheinlicher ein Konformationspaar (Diederwinkel 2-3 und 3-4) im Vergleich zum Erwartungswert der fünfdimensionalen Wahrscheinlichkeitsdichtefunktion ist, lässt sich über eine mathematische UND-Verknüpfung der beiden dreidimensionalen Wahrscheinlichkeitsdichtefunktionen erzielen (die beiden dreidimensionalen Wahrscheinlichkeitsdichtefunktionen sind nicht unabhängig voneinander). Hierbei gilt:

$$\sigma = \log\!\left(MAX(PDF_{1234}) * MAX(PDF_{2345})\right) - \log\!\left(PDF_{1234}(psi_{23}, phi_{23}) * PDF_{2345}(psi_{34}, phi_{34})\right)$$

| | |
|---|---|
| $\sigma$: | gibt an, wieviel Zehnerpotenzen die gemessene Konformation unwahrscheinlicher ist, als die wahrscheinlichste Konformation ist |
| MAX: | Maximum einer Wahrscheinlichkeitsdichtefunktion |
| $PDF_{1234}$: | Wahrscheinlichkeitsdichtefunktion der Diederwinkel zwischen Aminosäure 2 und 3 des Tetrapeptides 1234, wobei 1234 Teil des Pentapeptids 12345 ist |
| $PDF_{2345}$: | Wahrscheinlichkeitsdichtefunktion der Diederwinkel zwischen Aminosäure 3 und 4 des Tetrapeptides 2345, wobei 2345 Teil des Pentapeptids 12345 ist |
| $psi_{23}$ / $psi_{34}$: | psi-Winkel zwischen Aminosäure 2 und 3 bzw. 3 und 4 aus dem Pentapeptid 12345 |
| $phi_{23}$ / $phi_{34}$: | phi-Winkel zwischen Aminosäure 2 und 3 bzw. 3 und 4 aus dem Pentapeptid 12345 |
| $PDF_{1234}(psi_{1234}, phi_{1234})$: | Wert der Wahrscheinlichkeitsdichtefunktion $PDF_{1234}$ für ein bestimmtes Wertepaar $(psi_{1234}, phi_{1234})$ |
| $PDF_{2345}(psi_{2345}, phi_{2345})$: | Wert der Wahrscheinlichkeitsdichtefunktion $PDF_{2345}$ für ein bestimmtes Wertepaar $(psi_{2345}, phi_{2345})$ |

Die Figuren 13A und 13B zeigen zunächst die Wahrscheinlichkeitsdichtefunktionen der Tetrapeptide ELRK bzw. LRKA. Es ist zu erkennen, dass beide Tetrapeptide im Vergleich zu den erlaubten Winkeln im Ramachandran-Diagramm nur noch sehr wenig verschiedene Konformationen einnehmen können, also gut bestimmte Funktionen mit hoher Stringenz sind. Die Figuren 13C und 13D zeigen die beiden dreidimensionalen Wahrscheinlichkeitsdichtefunktionen ELRK und LRKA, die sich aus dem Pentapeptid ELRKA ableiten lassen Man erkennt wiederum deutlich, dass diese beiden Wahrscheinlichkeitsdichtefunktionen, im Vergleich zu den in Figur 13A und 13B dargestellten Wahrscheinlichkeitsdichtefunktionen an Schärfe gewonnen haben. Für Figur 13C ergeben sich hierbei unter Berücksichtigung des Tetrapeptides ELRK nur noch *eine* mögliche psi/phi-Winkelkonformation. Gleiches gilt für das Tetrapeptid LRKA. An diesem Beispiel kann unmittelbar erkannt werden, dass die Diederwinkel nicht vollständig unabhängig voneinander gebildet werden, sondern diese in der durch die Wahrscheinlichkeitsdichtefunktionen beschriebenen Weise miteinander korrelierbar sind.

Die Figuren 14A und 14B zeigen die Wahrscheinlichkeitsdichtefunktionen der Tetrapeptide GAKA und AKAG. Auch in diesem Fall werden im Vergleich zum Ramachandran-Diagramm nur noch wenige mögliche Winkelkonformationen beobachtet. Die Figuren 14C und 14D zeigen die entsprechenden Tetrapeptide, GAKA und AKAG, die Teil des Pentapeptids GAKAG sind. Auffällig ist, dass im Vergleich zu den Wahrscheinlichkeitsdichtefunktionen in den Figuren 14A und 14B die möglichen Winkelkonformationen noch weiter eingeschränkt sind. Eine UND-Verknüpfung der beiden Wahrscheinlichkeitsdichtefunktionen bewirkt nun, dass für die benachbarten Diederwinkel jeweils nur noch zwei Konformationen möglich sind. Konkret bedeutet das für GAKAG nurmehr 150˚, -80˚ (psi) für GAKA und 140˚,-70˚(phi) für AKAG oder -40˚, -60˚ (psi) für GAKA und -40˚, -60˚ (phi) für AKAG möglich sind. Im Vergleich zu den Wahrscheinlichkeitsdichtefunktionen der Tetrapeptide ist somit ein zusätzlicher Informationsgewinn erfolgt, der sich beispielsweise zur Strukturmodellierung oder zur Verifizierung von Strukturinformationen verwenden lässt. Die Figur 15 verdeutlicht diesen Sachverhalt parallel noch einmal am Beispiel des Pentapeptids VILLE.

Das Beispiel zeigt, dass auch mit Oligopeptiden anderer Länge als vier, hier mit Oligopeptiden der Länge von fünf Aminosäuren, die entsprechenden Datenbanken und Listen mittels der beschriebenen mathematischen Verfahren etabliert werden können und eine Analyse und Strukturerstellung im Sinne der vorliegenden Erfindung auf Basis dieser Oligopeptidinformationen ermöglicht wird. Grundsätzlich können für die Erfindung alle Oligopeptidlängen von zwei Aminosäuren Sequenzlänge oder mehr verwendet werden.

In Kenntnis des beschriebenen Verfahrens ist es hiernach in effizienter Weise möglich, Konformationsmuster in Proteinen sicher zu erkennen und zu verändern. Dadurch werden auch neue Möglichkeiten im *de novo* Design und bei Punktmutationen von Proteinen eröffnet.

**Patentansprüche**

1. Computer-implementiertes Verfahren zur Validierung der Konformation gegebener aminosäurebasierter Moleküle, das die folgenden Schritte umfasst:

   a) Einteilen des aminosäurebasierten Moleküls in Oligopeptide gleicher Länge, wobei die Anzahl der Oligopeptide vorzugsweise durch die Formel:

$$n-(m-1)$$

   bestimmt ist, in der n die Aminosäureanzahl im aminosäurebasierten Molekül ist und m die Aminosäureanzahl im Oligopeptid ist,
   b) Ermitteln der sich zwischen zwei konkret benannten Aminosäuren befindenden psi- und phi-Winkel dieser Oligopeptide;
   c) Bereitstellen oder Erstellen einer Oligopeptiddatenbank, die Werte der phi- und psi-Winkel für diese Oligopeptide enthält;
   d) Berechnen von Wahrscheinlichkeitsdichtefunktionen der Konformationszustände dieser psi- und phi-Winkel aus den Datenbankinformationen;
   e) Ermitteln eines Erwartungswertes, der als das Maximum der Wahrscheinlichkeitsdichtefunktion der sich zwischen den zwei konkret benannten Aminosäuren befindenden psi- und phi-Winkel dieser Oligopeptide definiert wird;
   f) Subtrahieren des logarithmierten Erwartungswerts vom logarithmierten Wert der Wahrscheinlichkeitsdichtefunktion der in Schritt b) ermittelten psi- und phi-Winkelwerte;
   g) Aminosäure-positionsweise Auswertung der Differenzen, wobei, je kleiner die Differenz, desto größer die Wahrscheinlichkeit für die Richtigkeit der gegebenen Struktur ist.

2. Verfahren nach Anspruch 1, wobei in Schritt e) der Erwartungswert für Oligopeptide ermittelt wird, die gegenüber den im gegebenen aminosäurebasierten Molekül vorkommenden Oligopeptiden eine oder mehrere Aminosäuren oder Sequenzabschnitte bestimmter Länge in Form eines Ähnlichkeitsmaßstabes ausgetauscht aufweisen, wobei die Größe der Differenz zwischen dem logarithmierten Erwartungswert und dem logarithmierten Wert der Wahrscheinlichkeitsdichtefunktion der in Schritt b) ermittelten psi- und phi-Winkelwerte ein Maß für die durch den Austausch zu erwartende Konformationsänderung ist.

3. Verfahren nach Anspruch 1 oder 2, wobei die Oligopeptide jeweils aus fünf Aminosäuren bestehen (Pentapeptide).

4. Verfahren nach Anspruch 3, wobei die psi- und phi-Winkel zwischen der zweiten und dritten sowie dritten und vierten Aminosäure des Pentapeptids gemessen werden.

5. Verfahren nach Anspruch 1-2, wobei die Oligopeptide jeweils aus vier Aminosäuren bestehen (Tetrapeptide).

6. Verfahren nach Anspruch 5, wobei das Protein aus n Aminosäureresten besteht und die Zahl der Tetrapeptide n-3 beträgt.

7. Verfahren nach Anspruch 6, wobei die psi- und phi-Winkel zwischen der zweiten und dritten Aminosäure des Tetrapeptids gemessen werden.

8. Verfahren nach Anspruch 1, wobei eine gegebene aminosäurebasierte Struktur durch den Vergleich des logarithmierten Erwartungswertes und des logarithmierten Wertes der Wahrscheinlichkeitsdichtefunktion der in Schritt b) ermittelten psi- und phi-Winkelwerte bezüglich besonderer Eigenschaften bewertet wird.

**Claims**

1. Computer-implemented method for validating the conformation of given amino acid-based molecules, comprising the steps of:

   a) classifying the amino acid-based molecules in oligopeptides of the same length, wherein the number of oligopeptides is preferably determined by the formula:

$$n\text{-}(m\text{-}1)$$

   in which n is the number of amino acids in the amino acid-based molecule and m is the number of amino acids in the oligopeptide;
   b) determining the psi and phi angles of these oligopeptides that are present between two specified amino acids;
   c) providing or establishing an oligopeptide database which contains the values of the phi and psi angles of these oligopeptides;
   d) calculating probability-density functions of the conformational state of these psi and phi angles by using the database information;
   e) determining an expected value which will be defined the maximum value of the probability -density function of the psi and phi angles of these oligopeptides that are present between the two specified amino acids;
   f) subtracting the logarithmised expected value from the logarithmised value of the probability -density function of the psi and phi angles determined in step b);
   g) amino acid position-based evaluation of the differences, wherein the smaller the difference the higher the probability for the correctness of the structure given.

2. Method according to claim 1 wherein, in step e), the expected value for oligopeptides is determined, wherein, in comparison with the oligopeptides present in the amino acid-based molecule given, one or more amino acids or sequence fragments of a defined length are replaced in form of a similarity scale, wherein the amount of difference between the logarithmised expected value and the logarithmised value of the probability -density function of the psi and phi angles determined within step b) is used as a degree for the conformational change to be expected due to the replacement.

3. Method according to claim 1 or 2, wherein the oligopeptides each consist of five amino acids (pentapeptides).

4. Method according to claim 3, wherein the psi and phi angles between the second and third as well as between the third and fourth amino acid of the pentapeptide are measured.

5. Method according to claim 1 to 2, wherein the oligopeptides each consist of four amino acids (tetrapeptides).

6. Method according to claim 5, wherein the protein consists of n amino acid residues and the number of tetrapeptides is n-3.

7. Method according to claim 6, wherein the psi and phi angles between the second and the third amino acid of the tetrapeptide are measured.

8. Method according to claim 1, wherein, with regard to special properties, a given amino acid-based structure is evaluated by comparing the logarithmised expected value with a logarithmised value of the probability -density function of the psi and phi angle values determined in step b).

**Revendications**

1. Procédé installé sur ordinateur pour la validation de la conformation de molécules données basées sur des aminoacides, comprenant les étapes suivantes consistant à :

   a) diviser la molécule basée sur des aminoacides en oligopeptides de longueur identique, le nombre des oligopeptides étant de préférence donné par la formule :

$$n-(m-1)$$

dans laquelle n est le nombre d'aminoacides dans la molécule basée sur des aminoacides et m le nombre d'aminoacides dans l'oligopeptide ;

b) déterminer les angles psi et phi, compris entre deux aminoacides concrètement nommés, de ces oligopeptides ;

c) préparer ou créer une base de données d'oligopeptides qui contient des valeurs des angles phi et psi pour ces oligopeptides ;

d) calculer des fonctions de densité de probabilité des états de conformation de ces angles psi et phi à partir des informations de la base de données ;

e) déterminer une valeur escomptée qui est définie comme étant le maximum de la fonction de densité de probabilité des angles psi et phi, compris entre deux aminoacides concrètement nommés, de ces oligopeptides ;

f) soustraire le logarithme de la valeur escomptée du logarithme de la valeur de la fonction de densité de probabilité des valeurs angulaires psi et phi déterminées à l'étape b) ;

g) évaluer les différences eu égard à la position des aminoacides, sachant que plus la différence est petite plus la probabilité de la justesse de la structure donnée est grande.

2. Procédé selon la revendication 1, selon lequel on détermine à l'étape e) la valeur escomptée pour des oligopeptides qui comportent, échangés par rapport à des oligopeptides présents dans la molécule donnée basée sur des aminoacides, un ou plusieurs aminoacides ou des tronçons de séquence d'une certaine longueur sous forme d'échelle de similitude, la grandeur de la différence entre le logarithme de la valeur escomptée et le logarithme de la valeur de la fonction de densité de probabilité des valeurs angulaires psi et phi déterminées à l'étape b) étant une mesure de la variation de conformation à laquelle il faut s'attendre suite à l'échange.

3. Procédé selon la revendication 1 ou 2, selon lequel les oligopeptides sont constitués à chaque fois de cinq aminoacides (pentapeptides).

4. Procédé selon la revendication 3, selon lequel les angles psi et phi sont mesurés entre le deuxième et le troisième ainsi qu'entre le troisième et le quatrième aminoacide du pentapeptide.

5. Procédé selon la revendication 1 ou 2, selon lequel les oligopeptides sont constitués à chaque fois de quatre aminoacides (tétrapeptides).

6. Procédé selon la revendication 5, selon lequel la protéine est constituée de n résidus aminoacides et selon lequel le nombre des tétrapeptides est égal à n-3.

7. Procédé selon la revendication 6, selon lequel les angles psi et phi sont mesurés entre le deuxième et le troisième aminoacide du tétrapeptide.

8. Procédé selon la revendication 1, selon lequel on évalue des propriétés particulières d'une structure donnée basée sur des aminoacides en comparant le logarithme de la valeur escomptée et le logarithme de la valeur de la fonction de densité de probabilité des valeurs angulaires psi et phi déterminées à l'étape b).

**Figur 1:** Definition der verwendeten Winkel (A) und Ramachandran-Diagramm (B).

**Figur 2:** Kerndichteschätzungen (schematische Darstellung).

**Figur 3:** Wahrscheinlichkeitsdichtefunktion für das beispielhaft ausgewählte Tetrapeptid EALC (Glutamat, Alanin, Leucin, Cystein).

**Figur 4:** Wahrscheinlichkeitsdichtefunktionen für das Tetrapeptid ACNE (A), ACNK (B), und ACNG (C).

EP 1 513 092 B1

**Figur 5:** Wahrscheinlichkeitsdichtefunktionen für das Tetrapeptid CIDL (A) und CIDV (B).

**Figur 6:** Validierung für die beiden nativen Proteinstrukturen Bacterioferritin (PDB Code 1BCF, Untereinheit A1; Figur A) und UDP-N-Acetylglucosamin-Acyltransferase (PDB Code 1LXA, Figur B).

**Figur 7:** Validierung für die nichtnative Proteinstrukturen Bacterioferritin (PDB Code 1BCF, Untereinheit A1) transformiert nach UDP-N-Acetylglucosamin-Acyltransferase (PDB Code 1LXA; Figur A) und die nichtnative Proteinstruktur UDP-N-Acetylglucosamin-Acyltransferase (PDB Code 1LXA) transformiert nach Bacterioferritin (PDB Code 1BCF, Untereinheit A1; Figur B).

**Figur 8:** Sequenz-Struktur Alignment der δ5-3-Ketosteroid-Isomerase (PDB Code 8CHO). A: Winkelabweichungen nicht erlaubt. B: Winkelabweichungen erlaubt. C: Kristallstruktur des Proteins.

**Figur 9:** Sequenz-Struktur-Alignment des Proteins Ferrocytochrom C (PDB Code 1CYC). A: Winkelabweichungen nicht erlaubt. B: Winkelabweichungen erlaubt. C: Kristallstruktur des Proteins.

**Figur 10:** Sequenz-Struktur-Alignment von δ5-3-Ketosteroid-Isomerase gegen die Struktur von Ferrocytochrom C. A: Winkelabweichungen nicht erlaubt. B: Winkelabweichungen erlaubt.

**Figur 11:** Sequenz-Struktur-Alignment des ribosomalen Proteins L30E (PDB Code 1H7M). A: Die Wahrscheinlichkeitsdichtefunktionen erlauben keine Winkelabweichnungen. B: Die Wahrscheinlichkeitsdichtefunktionen erlauben Winkelabweichungen. C: Die Kristallstruktur des Proteins.

**Figur 12:** Sequenz-Struktur-Alignment des Proteins yajq (PDB Code 1IN0). A: Die Wahrscheinlichkeitsdichtefunktionen erlauben keine Winkelabweichnungen. B: Die Wahrscheinlichkeitsdichtefunktionen erlauben Winkelabweichungen. C: Die Kristallstruktur des Proteins.

EP 1 513 092 B1

**Figur 13:** Wahrscheinlichkeitsdichtefunktion für das Tetrapeptid ELRK (A) und LRKA (B), sowie für das Tetrapeptid ELRK aus dem Pentapeptid ELRKA (C) und das Tetrapeptid LRKA aus dem Pentapeptid ELRKA (D).

**Figur 14:** Wahrscheinlichkeitsdichtefunktion für das Tetrapeptid GAKA (A) und AKAG (B), sowie für das Tetrapeptid GAKA aus dem Pentapeptid GAKAG (C) und das Tetrapeptid AKAG aus dem Pentapeptid GAKAG (D).

**Figur 15:** Wahrscheinlichkeitsdichtefunktion für das Tetrapeptid VILL (A) und ILLE (B), sowie für das Tetrapeptid VILL aus dem Pentapeptid VILLE (C) und das Tetrapeptid ILLE aus dem Pentapeptid VILLE (D).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **Böhm.** *Biophys. Chem.,* 1996, vol. 59, 1-32
- **Berman et al.** *Nature Struct. Biol.,* 2000, vol. 7, 957-959
- **Hilbert et al.** *Proteins: Struct. Funct. Genet.,* 1993, vol. 7, 138-151
- **McGuffin ; Jones.** *Proteins: Struct.Funct.Genet.,* 2003, vol. 52, 166-175
- **Garnier et al.** *J. Mol. Biol.,* 1978, vol. 120, 97-120
- **Kabsch ; Sander.** *Biopolymers,* 1983, vol. 22, 2577-2637
- **Rooman ; Wodak.** *Nature,* 1988, vol. 335, 45-49
- **Anfinsen ; Scheraga.** *Adv. Protein Chem. Bd.,* 1975, vol. 29, 205-300
- **Jaenicke.** *Prog. Biophys. Mol. Biol.,* 1987, vol. 49, 117-237
- **Jaenicke.** *Naturwissenschaften,* 1988, vol. 75, 604-610
- **Hardin et al.** *Curr. Opin. Struct. Biol.,* 2002, vol. 12, 176-181
- **Novotny et al.** *Proteins,* 1988, vol. 4, 19-30
- **Bowie et al.** *Science,* 1991, vol. 253, 164-170
- *Protein Engineering,* 1997, vol. 10, 1155-1162
- **Hong Seok Kang et al.** *J. Mol. Biol.,* 1993, vol. 229, 448-460
- **Ulrich Krengel.** Einführung in die Wahrscheinlichkeitstheorie und Statistik. Vieweg Verlag, 2003, vol. 7
- **Needleman, S. B. ; Wunsch, C. D.** *J.Mol.Biol.,* 1970, vol. 48, 443-453
- **Gapstrafen ; Gotoh.** *J. Mol. Biol.,* 1982, vol. 162, 705-708
- **Pearson.** *Methods Enzymol.,* 1996, vol. 266, 227-258
- **Adrian W. Bowman ; Adelchi Azzalini.** Applied Smoothig Techniques for Data Analysis. *Oxford Statistical Science,* vol. 18
- **Kabsch ; Sander.** *Proc. Natl. Acad. Sci. U.S.A.,* 1984, vol. 81, 1075-1078
- **Moult et al.** Struct. Funct. Genet. *Proteins,* 1999, vol. 3